# FASCICULE DE BREVET EUROPEEN

(11) **EP 2 825 531 B1**
(45) Date de publication et mention de la délivrance du brevet: **19.12.2018**
(21) Numéro de dépôt: 13720075.4
(22) Date de dépôt: 14.03.2013
(51) Int. Cl.: C07D 307/62, A61K 8/67, A61K 8/37, A61Q 19/08, A61K 8/02

(54) **CONJUGUÉS DE VITAMINE C**
VITAMIN C KONJUGATE
VITAMINE C CONJUGATES

(30) Priorité: 16.03.2012 FR 1252382
(43) Date de publication de la demande: 21.01.2015
(73) Titulaire: Centre National de la Recherche Scientifique (C.N.R.S.), 75794 Paris Cedex 16 (FR); UNIVERSITE PARIS-SUD (PARIS XI), 91405 Orsay Cédex (FR)
(72) Inventeur: COUVREUR, Patrick, 91140 Villebon Sur Yvette (FR); ZOUHIRI, Fatima, 92290 Chatenay-Malabry (FR); GREF, Ruxandra, 91370 Verrieres Le Buisson (FR); DESMAELE, Didier, 94260 Fresnes (FR)
(74) Mandataire: IPAZ
(86) Numéro de dépôt international: PCT/IB2013/052031
(87) Numéro de publication internationale: WO 2013/136294

(56) Documents cités:
- EP-A2- 0 296 483
- WO-A1-2006/019186
- JP-A- 2012 001 457
- COUVREUR PATRICK ET AL: "Squalenoyl nanomedicines as potential therapeutics", NANO LETTERS, ACS, US, vol. 6, no. 11, 10 juillet 2006 (2006-07-10), pages 2544-2548, XP002489419, ISSN: 1530-6984, DOI: 10.1021/NL061942Q [extrait le 2006-10-07] cité dans la demande
- TROMMER H ET AL: "Overcoming the Stratum Corneum: The Modulation of Skin Penetration", SKIN PHARMACOLOGY AND PHYSIOLOGY: JOURNAL OF PHARMACOLOGICAL ANDBIOPHYSICAL RESEARCH, S. KARGER AG, BASEL, CH, vol. 19, no. 2, 9 mai 2006 (2006-05-09), pages 106-121, XP002580800, ISSN: 1660-5527, DOI: 10.1159/000091978 [extrait le 2006-05-09]
- BEKKARA-AOUNALLAH F ET AL: "Novel PEGylated nanoassemblies made of self-assembled squalenoyl nucleoside analogues", ADVANCED FUNCTIONAL MATERIALS, WILEY - V C H VERLAG GMBH & CO. KGAA, DE, vol. 18, no. 22, 24 novembre 2008 (2008-11-24), pages 3715-3725, XP009117031, ISSN: 1616-301X, DOI: 10.1002/ADFM.200800705

## Description

La présente invention vise à proposer un nouveau bioconjugué de la vitamine C particulièrement intéressant pour son activité à l'égard de la peau. La présente invention concerne également des procédés pour sa préparation, des compositions le comprenant ainsi que son utilisation dans les domaines cosmétique, dermatologique, pharmaceutique et alimentaire.

La peau humaine est constituée de deux compartiments à savoir un compartiment superficiel, l'épiderme, et un compartiment profond, le derme, qui fournit à l'épiderme son support solide.

Ce derme, tissu nourricier de l'épiderme, est principalement constitué de fibroblastes et d'une matrice extracellulaire composée elle-même principalement de collagène, d'élastine et d'une substance, dite substance fondamentale, composants synthétisés par le fibroblaste.

Les fibroblastes synthétisent majoritairement des collagènes, des glycoprotéines matricielles autres que les collagènes (fibronectine, laminine), des protéoglycannes et de l'élastine. Les kératinocytes synthétisent majoritairement des GAGs sulfatés et de l'acide hyaluronique.

Quant à la matrice extracellulaire du derme, elle est comme celle de tous les tissus conjonctifs de l'organisme, composée de protéines appartenant à plusieurs grandes familles : les collagènes, les glycoprotéines matricielles autres que les collagènes (fibronectine, laminine), l'élastine et les protéoglycannes (PGs). On trouve également des glycosaminoglycannes (GAGs) sous forme libre (c'est à dire non liés à une protéine).

Les composants de la matrice extracellulaire jouent notamment un rôle important dans les propriétés mécaniques de la peau, en particulier dans sa tonicité, sa fermeté, son élasticité et sa souplesse.

Le collagène est une protéine composée de trois chaînes alpha polypeptidiques reliées par des liaisons hydrogène entre l'hydroxylysine et l'hydroxyproline et des liaisons covalentes associées. Selon les diverses combinaisons possibles, il existe plusieurs types de collagène avec des structures qui leur sont propres, se retrouvant dans des organes particuliers.

Parmi ces types de collagène, on peut notamment citer le collagène de type I et le collagène de type III qui sont localisés majoritairement au sein du derme. Le collagène de type I participe notamment à la rigidité et à la résistance des tissus. Au cours du vieillissement, il est de plus en plus prédominant (plus de 80 %). Ainsi, les fibres deviennent plus rigides et moins souples, entrainant une perte de la fermeté de la peau.

Quant au collagène de type III, également nommé « collagène de jeunesse », il est synthétisé en abondance au cours de la vie foetale et de l'adolescence.

Cependant, il a été constaté qu'avec l'âge, la quantité de collagène de type III diminue au profit du collagène de type I (Wang et al., African J. Biotechn., 2011, vol 10(13),2524-2529).

Par ailleurs, on sait que la vitamine C, également connue sous le nom d'acide ascorbique, de 5-(1,2-dihydroxy-éthyl)-3,4-dihydroxy-5H-furan-2-one ou encore de 3-céto-L-gulofuranolactone, est impliquée dans des centaines de processus dans l'organisme.

On sait également que la vitamine C a notamment pour fonction d'aider le corps à fabriquer le collagène, essentiel à la formation du tissu conjonctif de la peau, des ligaments et des os.

D'une manière générale, un organisme est supplémenté en vitamine C par voie orale, classiquement via l'alimentation et de manière plus occasionnelle via des compléments alimentaires enrichis en vitamine C. Plus récemment, il a été proposé d'administrer par voie topique la vitamine C, notamment à des fins d'amélioration de l'aspect esthétique de la peau.

Malheureusement, la vitamine C est également connue pour être l'une des vitamines les plus fragiles. Ainsi, elle est sensible à la chaleur, craint les milieux basiques et surtout est très sensible à l'oxygène de l'air.

Par conséquent, la plupart des formulations existantes comprenant de la vitamine C doivent généralement contenir des agents stabilisants ou des conservateurs afin d'éviter toute dégradation de celle-ci avant son utilisation.

En plus, la vitamine C en tant que telle possède un faible pouvoir de pénétration dans la peau du fait notamment de son caractère hydrophile.

Pour suppléer ce défaut de perméabilité de l'acide ascorbique dans l'épiderme et le derme de la peau, la demande internationale WO 2006/019186 propose de mettre en oeuvre dans des produits de soin par voie topique et des produits alimentaires par voie orale des dérivés liposolubles comprenant de la vitamine C sous une forme couplée en sa position 6 à un acide gras polyinsaturé (6-O-PUFA). D'autres dérivés de la vitamine C comme les esters d'acides carboxyliques aliphatiques en C₂-C₂₀ en position 6 de l'acide ascorbique tels que le 6-ascorbyl palmitate et le 6-ascorbyl linolate sont également décrits dans le brevet US 4,997,958 mais à des fins d'antioxydants.

Toutefois, et comme il ressort des essais figurant ci-après, des dérivés tels que décrits dans les documents WO 2006/019186 et US 4,997,958 ne s'avèrent pas totalement satisfaisants en terme de pénétration dans les couches profondes de la peau.

Le document Couvreur et al. (Couvreur et al., NANO LETTERS, ACS, US, vol. 6, no. 11, pages 2544 - 2548) divulgue des nanoparticules à base d'esters de l'acide scalénique comme agent anticancéreux ou antiviral.

Le document Trommer et al. (Trommer et al., Skin pharmacology and physiology : Journal of pharmacological and biophysical research, S. KARGER AG, BASEL, CH, vol 19, no.2, 9 mai 2006, pages 106-121) divulgue des esters de l'acide ascorbiques avec des acides gras en tant qu'antioxydants dans les produits alimentaires.

Le document Bekkara - Aounallah et al. (Bekkara - Aounallah et al., Advanced functional materials, Wiley - VCH Verlag GMBH & Co. KGAA, DE, vol.18, no 22, 24 novembre 2008, pages 3715-3725) divulgue des nanoparticules à base d'esters de l'acide squalénique comme agent anticancéreux.

La demande de brevet EP0296483 divulgue un procédé de préparation d'esters en 6 de l'acide ascorbique.

La demande de brevet JP2012001457 divulgue un agent antirides comprenant un ester de l'acide L-ascorbique 2-phosphate.

En outre, ces dérivés déjà connus ne donnent pas entière satisfaction sur le plan de la cytotoxicité et certains d'entre eux ne se prêtent pas toujours à une formulation à concentration élevée. Par exemple, le composé 6-ascorbyl palmitate formulé dans une huile ne se présente plus, au-delà de 5% en poids, exprimé en poids d'actif vitamine C par rapport au poids total de la composition, sous la forme d'une formulation homogène et a tendance à cristalliser. Ces inconvénients rendent ce composé particulièrement peu efficace dans une formulation huileuse qui est pour des raisons évidentes, une formule galénique particulièrement appréciée pour une administration par voie topique.

La présente invention a précisément pour objet de proposer de nouveaux dérivés de la vitamine C donnant satisfaction en ces termes.

Ainsi, selon un de ses aspects, la présente invention vise à fournir de nouveaux dérivés de vitamine C qui pénètrent plus facilement dans la peau et qui permettent d'améliorer l'effet bénéfique de la vitamine C dans les couches profondes de la peau.

Selon un autre de ses aspects, la présente invention vise à proposer de nouveaux dérivés de vitamine C capables de présenter une stabilité accrue à l'égard de l'oxydation.

Selon encore un autre de ses aspects, la présente invention vise à proposer de nouveaux dérivés de vitamine C aptes à stimuler l'expression du collagène, et plus particulièrement du collagène III.

Selon encore un autre de ses aspects, la présente invention vise à fournir de nouveaux dérivés de vitamine C aptes à stimuler l'expression des GAGs.

Selon encore un autre de ses aspects, la présente invention vise à proposer de nouveaux dérivés de vitamine C, propices à une formulation aussi bien dans un milieu aqueux que dans un milieu huileux, aptes à être formulés de manière homogène, et ce même pour des quantités importantes en équivalent de vitamine C.

Selon encore un autre de ses aspects, la présente invention vise à proposer de nouveaux dérivés de vitamine C compatibles avec tout mode d'administration et donnant satisfaction en terme d'innocuité.

Enfin selon un autre de ses aspects, la présente invention vise à fournir de nouveaux dérivés de vitamine C capables d'améliorer la morphologie générale de la peau en termes de souplesse, d'hydratation, d'épaisseur, d'élasticité.

Contre toute attente, les inventeurs de la présente invention ont constaté que le couplage covalent de l'acide ascorbique avec au moins une molécule spécifique permet précisément de donner satisfaction en ces termes.

Ainsi, un objet de la présente invention concerne un conjugué formé d'au moins une molécule de 5-(1,2-dihydroxy-éthyl)-3,4-dihydroxy-5H-furan-2-one ou dérivé liée de manière covalente à au moins un radical hydrocarboné de formule (A) comme suit : 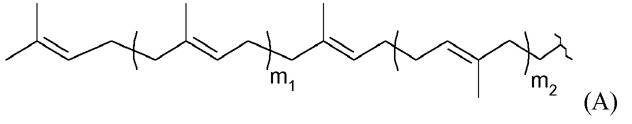 dans laquelle :
- m₁ = 0, 1,2,3,4,5 ou 6 ;
- m₂ = 0, 1,2,3,4,5 ou 6; et
- 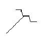 représente la liaison vers la molécule de 5-(1,2-dihydroxy-éthyl)-3,4-dihydroxy-5H-furan-2-one ou dérivé ayant un système 3,4-dihydroxy-5H-furan-2-one tel que schématisé comme suit :

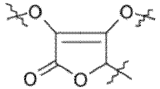

Au sens de l'invention, le terme complexe ou conjugué sera utilisé indifféremment pour désigner le produit de couplage entre au moins une molécule de 5-(1,2-dihydroxy-éthyl)-3,4-dihydroxy-5H-furan-2-one ou dérivé et au moins un radical hydrocarboné de formule (A).

Au sens de l'invention, le terme « liaison » associé au symbole 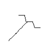 entend signifier que 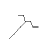 figure le site de liaison de l'entité de formule (A) à l'entité acide ascorbique ou dérivé. Ainsi, comme détaillé ci-après, le site de liaison peut être impliqué
soit directement dans une simple liaison covalente entre l'entité (A) et l'entité acide ascorbique ou dérivé, soit être figuré par une fonction résultant de l'intéraction entre deux fonctions réactives, par exemple entre une fonction carboxylique et une fonction alcool, ou encore être figuré par un groupement de type espaceur.

Ainsi, l'entité (A) et l'entité acide ascorbique ou dérivé peuvent être soit liées l'une à l'autre via une liaison covalente soit via un groupement espaceur ou une fonction de type ester, éther, phosphate ou amide, comme exposé ci-après.

Selon un autre objet, l'invention vise un conjugué tel que défini précédemment, dans lequel le composé ou radical hydrocarboné comprend de 12 à 40 atomes de carbone, de préférence de 18 à 30 atomes de carbone.

Comme exposé plus en détails ci-après, un tel composé ou radical hydrocarboné peut avantageusement dériver du squalène, du farnésol ou du géraniol et plus préférentiellement du squalène.

Avantageusement, les deux types d'entités formant le complexe défini précédemment sont couplées par une liaison covalente de type ester, éther, phosphate ou amide, et de préférence ester.

La présente invention a également pour objet une formulation huileuse comprenant un conjugué tel que décrit précédemment. Plus particulièrement, cette formulation se présente sous l'aspect d'un gel pouvant contenir jusqu'à au moins 3% en poids, voire au moins 10 % en poids, exprimé en poids d'actif vitamine C, par rapport au poids total de la composition.

Selon une variante préférée, l'huile considérée pour formuler le conjugué selon l'invention à l'état d'un tel gel est le squalène ou l'un de ses dérivés.

Selon un autre de ses aspects, la présente invention vise des nanoparticules d'un conjugué tel que décrit précédemment.

En effet, contre toute attente, les inventeurs ont constaté que le conjugué selon l'invention est capable de s'auto-assembler, c'est-à-dire à s'auto-organiser spontanément en milieu aqueux sous la forme de nanoparticules qui possèdent au moins les mêmes activités que le conjugué en tant que tel.

Ainsi, la présente invention concerne également un procédé de préparation desdites nanoparticules comprenant au moins la dispersion d'un conjugue selon la présente invention, dans au moins un solvant organique, à une concentration suffisante pour obtenir, lors de l'ajout du mélange correspondant, sous agitation, à une phase aqueuse, la formation instantanée de nanoparticules en suspension dans ladite phase aqueuse, et, le cas échéant, l'isolement desdites nanoparticules.

L'aptitude des conjugués selon l'invention, à s'organiser à l'état de nanoparticules conformes à la présente invention constitue une alternative avantageuse à plusieurs titres.

Tout d'abord, l'état nanoparticulaire du conjugue de la vitamine C selon l'invention permet avantageusement de formuler celle-ci pour être homogène de manière stabilisée en milieu aqueux.

Qui plus est, comme il ressort des essais exposés ci-après, l'état nanoparticulaire du conjugue de la vitamine C selon l'invention permet d'amplifier la pénétration de celle-ci jusqu'aux couches profondes de la peau. Cette amélioration de la pénétration dans les couches profondes de la peau permet avantageusement d'utiliser des concentrations moindres en vitamine C que celles usuellement requises.

En outre, les nanoparticules selon l'invention permettent avantageusement de stimuler l'expression du collagène et plus précisément du collagène III, ainsi que des GAGs, comme indiqué précédemment, et d'améliorer la morphologie générale de la peau.

En outre, compte tenu de la faible taille des particules de conjugués considérés selon l'invention, cette forme particulière est administrable sous la forme d'une suspension aqueuse par voie parentérale (ou injectable) et notamment par voie intraveineuse.

Ainsi, avantageusement, la taille moyenne de ces nanoparticules varie de 30 nm à 500 nm, en particulier de 40 à 250 nm, voire de 45 à 95 nm.

Des nanoparticules de conjugués formés par couplage d'au moins un radical hydrocarboné et, en particulier le squalène, avec un actif tel que la gemcitabine, les nucléosides, les acides nucléiques, les statines, les taxoïdes, la doxorubicine, l'épirubicine et la bêta-lactamine ont déjà été décrites dans les documents WO 2006/090029, Couvreur et al., Nano Lett. 2006, 6, pp. 2544-25 48, WO 2009/150344, WO 2009/071850, WO 2010/049899 et WO2010/049900.

Toutefois, ces actifs sont clairement très différents structurellement de la vitamine C.

La présente invention a également pour objet une composition cosmétique, dermatologique ou alimentaire comprenant à titre de matière active au moins un conjugué selon l'invention et/ou des nanoparticules selon l'invention, en association avec au moins un véhicule physiologiquement acceptable.

Selon un autre objet, l'invention concerne l'utilisation cosmétique d'un conjugué selon l'invention et/ou de nanoparticules selon l'invention pour la prévention et/ou le traitement des signes du vieillissement de la peau du corps et/ou du visage.

Par exemple, une composition cosmétique selon l'invention peut être utilisée pour la prévention et/ou le traitement des rides et/ou ridules, de la peau flétrie, du manque d'élasticité et/ou du tonus de la peau, de l'amincissement du derme, de la dégradation des fibres de collagène, de la peau molle, de la peau amincie et/ou des dégradations internes de la peau consécutives à une exposition aux rayonnements ultra-violets.

Selon un autre objet, la présente invention vise à protéger l'utilisation cosmétique d'un conjugué selon l'invention et/ou de nanoparticules selon l'invention pour stimuler la synthèse de collagène en particulier du collagène III et/ou stimuler la synthèse de glycosaminoglycanes.

La présente invention vise également un conjugué selon l'invention et/ou des nanoparticules selon l'invention pour son utilisation en médecine et plus précisément dans la prévention et/ou le traitement des brûlures et/ou des plaies et/ou de toute autre pathologie liée à la cicatrisation du derme et/ou de l'épiderme.

Encore selon un autre objet, la présente invention concerne un procédé de traitement cosmétique de la peau du corps et/ou du visage et/ou du cuir chevelu, dans lequel on administre une composition telle que définie précédemment.

### Complexe selon l'invention

Comme précisé précédemment, les conjugués selon l'invention sont formés par couplage d'une molécule de vitamine C ou analogue à au moins un radical hydrocarboné spécifique.

### Radical hydrocarboné

Au sens de la présente invention, le radical hydrocarboné répond à la formule (A) comme suit : 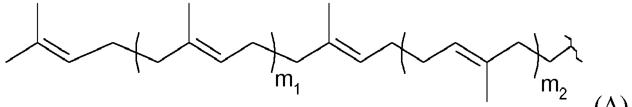 dans laquelle :
- m₁ = 0, 1, 2, 3, 4, 5 ou 6 ;
- m₂ = 0, 1, 2, 3, 4, 5 ou 6; et
- 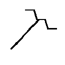 représente le site de liaison à la molécule la molécule de 5-(1,2-dihydroxy-éthyl)-3,4-dihydroxy-5H-furan-2-one ou dérivé ayant un système 3,4-dihydroxy-5H-furan-2-one tel que schématisé comme suit :

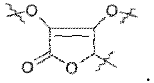

Ce radical hydrocarboné dérive généralement de la mise en présence d'au moins une molécule de composé de formule (A') : 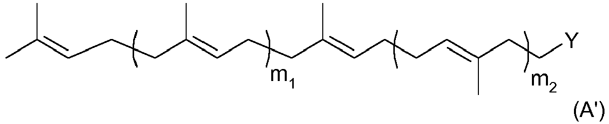 dans laquelle :
Y représente une fonction réactive et notamment de type alcool, acide carboxylique, ou phosphate ; et
m₁ et m₂ sont tels que définis pour le radical de formule (A).
Le composé hydrocarboné de formule (A') comprend au moins 12 atomes de carbone, en particulier de 12 à 40 atomes de carbone et de préférence de 18 à 30 atomes de carbone.

Avantageusement, les composés de formule (A') sont choisis parmi les composés de formule (A') dans lesquels :
- m₁ représente 1, m₂ représente 2 et Y est une fonction -COOH,
- m₁ et m₂ représentent 0 et Y est une fonction -COOH, et
- m₁ représente 1, m₂ représente 0 et Y est une fonction -COOH.

Plus précisément, un composé de formule (A) utile pour la formation d'un conjugué selon la présente invention est le squalène (encore appelé spiracène ou sirprène), qui est un intermédiaire essentiel de la biosynthèse du cholestérol.

Chimiquement, il est encore appelé (E) 2, 6, 10, 15, 19, 23-hexaméthyl-2, 6, 10, 14, 18, 22-tétracosahexène) de formule qui suit :

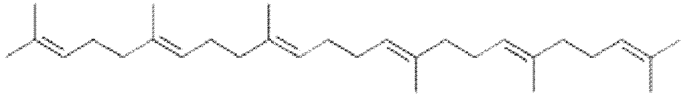

Dans ce mode de réalisation préféré de l'invention, le radical hydrocarboné présent dans un conjugué selon la présente invention est un radical de formule (A) dans laquelle m₁ = 1 et m₂ = 2.

Un autre composé utile pour la formation d'un complexe selon la présente invention est le farnésol qui est un alcool sesquiterpène acyclique.

Chimiquement, il est encore appelé (2E, 6E)-3,7,11-triméthyldodéca-2,6,10-trién-1-ol de formule qui suit :

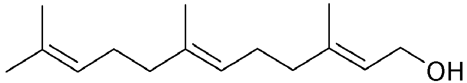

Dans cet autre mode de réalisation de l'invention, le radical hydrocarboné présent dans un complexe selon la présente invention est un radical de formule (A) dans laquelle m₁ = 1 et m₂ = 0.

Encore un autre composé utile pour la formation d'un conjugué selon la présente invention est le géraniol (également nommé rhodinol) qui est un alcool terpénique insaturé.

Chimiquement, il est encore appelé (2E)-3,7-diméthylocta-2,6-dién-1-ol de formule qui suit :

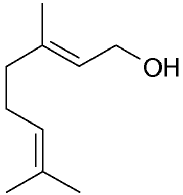

Dans cet autre mode de réalisation de l'invention, le radical hydrocarboné présent dans un complexe selon la présente invention est un radical de formule (A) dans laquelle m₁ = 0 et m₂ = 0.

Selon un mode de réalisation préféré de la présente invention, le dérivé hydrocarboné présent dans un complexe selon la présente invention est un dérivé squalénique.

A titre illustratif des composés hydrocarbonés aptes à former un complexe contenant au moins un dérivé squalénique selon la présente invention, on peut plus particulièrement citer l'acide squalénique et ses dérivés tel que l'acide 1, 1', 2-tris-norsqualénique, la 1, 1', 2-tris-norsqualénamine, le 1, 1', 2-tris-norsqualénol, le 1, 1', 2-tris-norsqualéthiol, l'acide squalénacétique, le squalényléthanol le squalényléthanethiol, la squalényléthylamine.

En particulier, un conjugué selon la présente invention pourra contenir au moins un radical dérivant du couplage covalent d'une molécule d'acide 1, 1', 2-tris-norsqualénique.

En particulier, un conjugué selon la présente invention pourra contenir au moins un radical dérivant du couplage covalent d'une molécule de 1, 1', 2-tris-norsqualénol.

Alternativement, un conjugué selon la présente invention peut comprendre au moins deux radicaux hydrocarbonés conformes à la présente invention.

### Vitamine C

Structurellement, la vitamine C et ses dérivés au sens de l'invention ont en commun un système 3,4-dihydroxy-5H-furan-2-one que l'on peu schématiser par le motif comme suit.

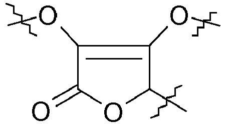

En conséquence, les conjugués selon l'invention possèdent avantageusement un tel motif.

Plus précisément, la vitamine C est figurée par la formule (IV) et sa forme tautomère comme suit.

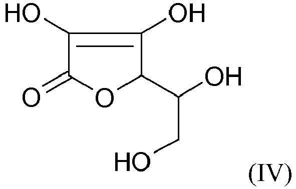

Les composés de formule générale (IV) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formules précitées peuvent exister à l'état d'acides ou de sels d'addition à des bases.

Ces sels sont avantageusement préparés avec des bases pharmaceutiquement acceptables, mais les sels d'autres bases utiles, par exemple, pour la purification ou la séparation des composés de formules précitées font également partie de l'invention.

Les composés de formule générale (IV) peuvent, en outre, se trouver sous forme d'hydrates ou de solvates, à savoir sous forme d'associations ou de combinaisons avec une ou plusieurs molécules d'eau ou avec un solvant. De tels hydrates et solvates font également partie de l'invention.

Avantageusement, selon la présente invention, on peut complexer l'acide ascorbique, l'ascorbate de sodium, l'ascorbate de potassium et/ou l'ascorbate de calcium, et préférentiellement l'acide ascorbique.

### Complexe vitamine C/radical hydrocarboné

Selon un mode de réalisation particulier, le conjugué conforme à l'invention est de formule générale (I) 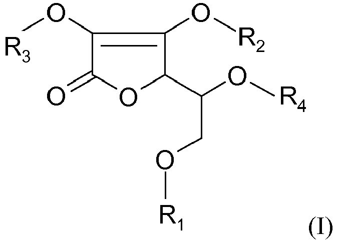 dans laquelle :
R₁, R₂, R₃ et R₄, identiques ou différents, représentent indépendamment l'un de l'autre un atome d'hydrogène ou un radical hydrocarboné de formule (A) tel que défini précédemment,
avec l'un au moins des groupements R₁, R₂, R₃ et R₄ étant différents de l'atome d'hydrogène.

Les dérivés de formule (I) selon l'invention dans lesquels R₂ est un atome d'hydrogène sont illustrés par la formule générale (I') qui suit : 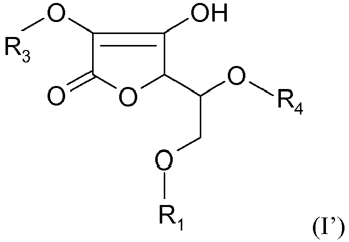 dans laquelle R₁, R₃ et R₄ sont tels que définis précédemment, l'un au moins des groupements R₁, R₃ et R₄ étant différents de l'atome d'hydrogène.

Selon un autre mode de réalisation particulier, le conjugué conforme à l'invention est de formule générale (IA) 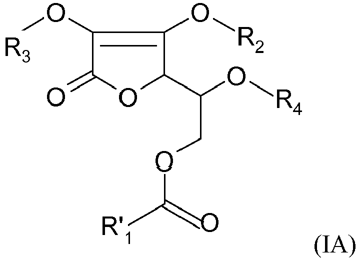 dans laquelle :
R₂, R₃ et R₄ sont tels que définis précédemment et R'₁ représente un radical hydrocarboné de formule (A) tel que défini précédemment.

Avantageusement, le conjugué selon l'invention est de formule générale (IA) dans laquelle R₂, R₃ et R₄ représentent tous trois un atome d'hydrogène.

Les radicaux hydrocarbonés selon l'invention sont généralement fixés par une liaison covalente au niveau du groupement hydroxyle porté par l'atome de carbone 2 et/ou 3 et/ou 4 et/ou 5 et/ou 6 de l'acide ascorbique, et de préférence au niveau du groupement hydroxyle porté par l'atome de carbone 6 de l'acide ascorbique, ou au niveau du groupement hydroxyle porté par l'atome de carbone 2 de l'acide ascorbique, ou au niveau du groupement hydroxyle porté par l'atome de carbone 3 de l'acide ascorbique.

Bien entendu, les complexes d'acide ascorbique selon la présente invention peuvent être des complexes comprenant deux dérivatisations, trois dérivatisations, voire quatre dérivatisations, celles-ci pouvant être identiques ou différentes.

Comme précisé ci-dessus, la formation du complexe vitamine C/radical hydrocarboné selon l'invention nécessite que les deux entités mise en présence portent respectivement une fonction dite réactive, c'est-à-dire susceptibles de former via leur interaction la liaison covalente attendue. Ces fonctions peuvent ou non être naturellement présentes sur les deux entités de départ. Dans la négative, l'entité de départ devra subir une modification, préalablement à la réaction de couplage.

Plus précisément, le composé hydrocarboné selon l'invention est généralement porteur d'une fonction susceptible de réagir avec une fonction présente sur la molécule de vitamine C considérée, de manière à établir un lien covalent entre les deux entités, par exemple de type ester, éther, phosphate ou amide, formant ainsi un complexe covalent.

Avantageusement, il s'agit d'une fonction ester. Par exemple, le composé hydrocarboné apte à réagir avec une molécule de vitamine C ou l'un de ses dérivés pour former le complexe précité, est l'acide 1,1',2-tris-norsqualénique ou l'un de ses dérivés comme par exemple son halogénure d'acide et plus particulièrement le chlorure d'acide, ou son anhydride mixte avec le chloroformiate d'éthyle. Préférentiellement, le chlorure d'acide dérivé de l'acide 1,1',2-tris-norsqualénique est utilisé.

Selon une autre variante, il s'agit d'une fonction éther. Par exemple, le composé hydrocarboné apte à réagir avec une molécule de vitamine C ou l'un de ses dérivés pour former le conjugué précité, est le 1,1',2-tris-norsqualénol ou l'un de ses dérivés comme par exemple le mésylate de 1,1',2-tris-norsqualénol.

Selon une autre variante de réalisation, le lien covalent existant entre les deux types de molécules peut être figuré par un espaceur fonctionnel ou encore bras de liaison. Un tel bras peut notamment s'avérer utile pour augmenter la force de l'interaction vitamine C/radical hydrocarboné.

Un tel bras peut également être avantageux pour introduire via chacune des deux extrémités de son squelette les fonctions adéquates, c'est-à-dire possédant respectivement l'affinité réactionnelle attendue, l'une pour la fonction présente sur le composé à structure hydrocarbonée selon l'invention, et l'autre pour la fonction présente sur la molécule vitamine C considérée.

On peut également envisager que ce bras de liaison possède en outre au niveau de son squelette une fonction labile, propice ultérieurement à la séparation du composé à structure hydrocarbonée de la molécule de vitamine C considérée. Il peut par exemple s'agir d'un motif peptidique reconnaissable par une enzyme.

Les motifs de type bras de liaison sont bien connus de l'homme de l'art et leur mise en oeuvre relève clairement de ses compétences.

A titre représentatif des bras de liaison envisageables selon l'invention, on peut notamment citer les chaînes alkylène, les motifs (poly)aminoacides, polyols, saccharidiques, et polyéthylèneglycol (polyétheroxides).

Au sens de la présente invention on entend par :
- « chaînes alkylène », un groupe C₁-C₄ alkylène c'est-à-dire un groupe alkyle divalent pouvant comprendre de 1 à 4 atomes de carbone. A titre d'exemple, on peut citer le méthylène, le propylène, l'isopropylène, le butylène.
- « motif saccharidique », un radical comprenant au moins un radical choisi parmi les trioses (glycéraldéhyde, dihydroxyacétone), tétroses (érythrose, thréose, érythrulose,), pentoses (arabinose, lyxose, ribose, désoxyribose, xylose, ribulose, xylulose), hexoses (allose, altrose, galactose, glucose, gulose, idose, mannose, talose, fructose, psicose, sorbose, tagatose), heptoses (mannoheptulose, sedoheptulose), octose (octolose, 2-céto-3-désoxy-manno-octonate), isonoses (sialose), et
- « motif (poly)aminoacide », un motif ayant au moins une unité : 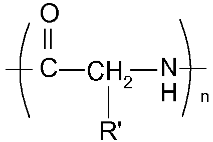 dans laquelle n est supérieur ou égal à 1 et R' représente un atome d'hydrogène, un groupe C₁₋₆ alkyle, éventuellement substitué par un ou plusieurs hydroxyles, un C₁₋₆ alcoxy.

Ainsi, au sens de la présente invention, un « lien covalent » figure de préférence une liaison covalente notamment telle que précisée ci-dessus, mais couvre également un lien covalent figuré par un bras de liaison tel que défini précédemment.

La réaction de couplage nécessaire à l'établissement d'au moins une liaison covalente entre au moins une molécule de vitamine C considérée et au moins un radical hydrocarboné conforme à la présente invention, peut être effectuée selon des conditions standards et sa réalisation relève donc clairement des connaissances de l'homme de l'art.

Cette réaction est généralement réalisée en solution en présence et en excès d'au moins un composé hydrocarboné considéré selon la présente invention par rapport à la molécule de vitamine C mise en oeuvre selon l'invention, par exemple à raison de deux équivalents, selon les conditions standards requises pour faire interagir les deux fonctions spécifiques portées par chacune des deux entités.

De préférence, un composé hydrocarboné de départ pour la synthèse d'un complexe selon l'invention est un dérivé squalénique sous forme acide, tel que par exemple l'acide 1,1',2-tris-norsqualénique, pouvant être préparé selon le procédé décrit à l'exemple la.

Ensuite, le couplage covalent des deux entités du complexe conforme à l'invention peut être notamment réalisé comme suit.

Selon un autre objet, la présente invention se rapporte à un procédé de préparation (ci-après nommé première voie) du complexe selon l'invention comprenant :
la condensation d'au moins une molécule d'un halogénure d'acyle de formule (III) 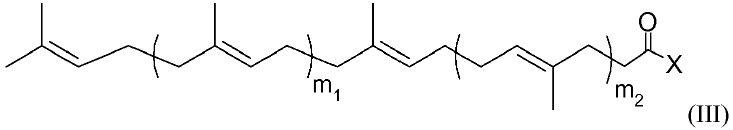 dans laquelle X est un atome d'halogène et de préférence un atome de chlore, et m₁ et m₂ sont tels que définis précédemment dans le composé de formule (A),
et d'au moins une molécule de 5-(1,2-dihydroxy-éthyl)-3,4-dihydroxy-5H-furan-2-one de formule (IV) 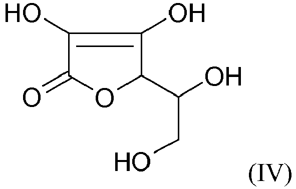 pour obtenir ledit complexe ou conjugué selon l'invention.

La préparation de l'halogénure d'acyle de formule (III) à partir de l'acide carboxylique correspondant et d'un agent halogénant tel que le chlorure de thionyle SOCl₂, le trichlorure de phosphore PCl₃, le pentachlorure de phosphore PCl₅ et le chlorure d'oxalyle, relève clairement des connaissances générales de l'homme du métier.

La première voie de préparation du conjugué selon l'invention permet d'obtenir des quantités dudit complexe avec un rendement d'au moins 30%, voire d'au moins 34%, et avec un degré de pureté d'environ 90 %.

Selon un autre objet, l'invention concerne un procédé de préparation (ci-après nommé seconde voie) du conjugué selon l'invention comprenant la réaction d'estérification entre au moins une molécule de 5-(1,2-dihydroxy-éthyl)-3,4-dihydroxy-5H-furan-2-one de formule (IV) 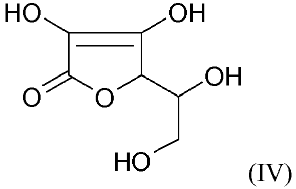 et au moins une molécule d'un acide de formule (II) 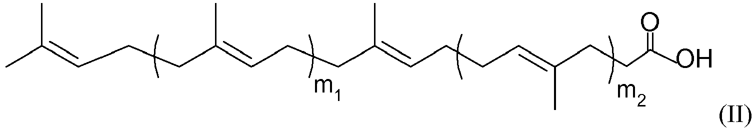 dans laquelle m₁ et m₂ sont tels que définis précédemment pour le composé de formule (A).

Avantageusement, ladite réaction d'estérification est réalisée en présence d'une lipase à titre de catalyseur.

Les lipases ou triacylglycérol acyl-hydrolases sont des enzymes atypiques de par leur mécanisme d'action et leur spécificité de substrats (Fickers et al. Biotechnol. Agron. Soc. Environ. 2008, 12(2), 119-130 ou Alloue et al., Biotechnol. Agron. Soc. Environ. 2008, 12(1), 57-68). Elles sont des catalyseurs très largement utilisées en synthèse organique, principalement en raison de leur stabilité et de leur activité en milieu solvant. Parmi les lipases conformes à l'invention, on peut citer notamment les lipases végétales, les lipases de mammifères et les lipases microbiennes. Selon l'invention, on préfèrera utiliser une lipase microbienne, encore plus préférentiellement, une lipase choisie parmi C. cylindracea, C. antarctica, C. miehei et leurs mélanges, et de manière encore plus préférée une lipase C. antarctica telle que celle commercialisée par la société Novozyme Corp sous la dénomination commerciale Novozyme 435®.

La seconde voie de préparation du conjugué selon l'invention conduit à l'obtention de quantités dudit complexe avec un rendement inférieur ou égal à 30 % et avec un degré de pureté supérieur ou égal à 95 %.

Selon un autre objet, la présente invention se rapporte à un procédé de préparation du conjugué selon l'invention comprenant :
la réaction entre au moins une molécule de 5-(1,2-dihydroxy-éthyl)-3,4-dihydroxy-5H-furan-2-one de formule (IV') 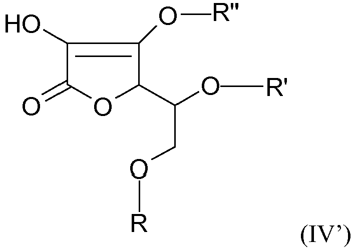 dans laquelle R, R' et R" représentent un atome d'hydrogène ou un groupement protecteur ;
et au moins une molécule d'un composé de formule (VIII) 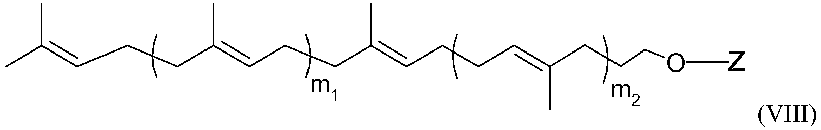 dans laquelle m₁ et m₂ sont tels que définis précédemment pour le composé de formule (A); et Z est un atome d'hydrogène ou un groupe -SO₂-CH₃.

Selon un mode de réalisation préféré, le composé hydrocarboné de départ pour la synthèse du conjugué selon l'invention est le 1,1',2-trisnorsqualénol comme notamment illustré dans l'exemple 1b.

Ensuite le couplage covalent des deux entités du conjugué conforme à l'invention peut être notamment réalisé comme suit.

Un procédé de préparation du conjugué selon l'invention comprenant la réaction d'au moins une molécule d'alcool de formule (V) 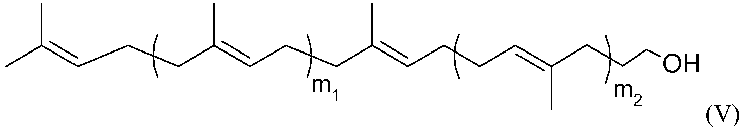 dans laquelle m1 et m2 sont tels que définis précédemment,
avec au moins une molécule dérivée de l'acide ascorbique de formule (VI) 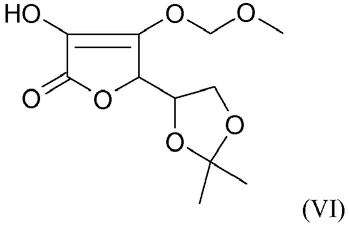 suivie d'une hydrolyse pour obtenir ledit conjugué selon l'invention, est également décrit.

Selon encore un autre mode de réalisation préféré, le composé hydrocarboné de départ pour la synthèse du conjugué selon l'invention est le mésylate de 1,1',2-trisnorsqualénol comme illustré notamment dans l'exemple 1c.

Ensuite le couplage covalent des deux entités du conjugué conforme à l'invention peut être notamment réalisé comme suit.

Un procédé de préparation du conjugué selon l'invention comprenant la réaction d'au moins une molécule de mésylate d'alcool de formule (VII) 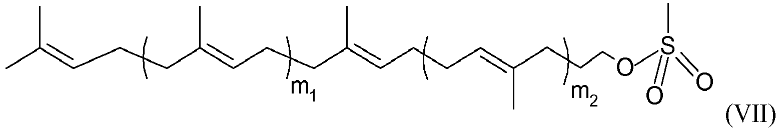 dans laquelle m₁ et m₂ sont tels que définis précédemment,
avec au moins une molécule de 5-(1,2-dihydroxy-éthyl)-3,4-dihydroxy-5H-furan-2-one de formule (IV) 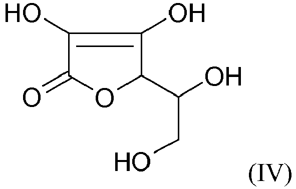 pour obtenir ledit conjugué selon l'invention, est également décrit.

### Gel selon l'invention

Comme indiqué précédemment, la présente invention a également pour objet un conjugué selon l'invention formulé à l'état de gel avec au moins une huile.

Parmi les huiles convenant à la présente invention, on peut citer plus particulièrement le squalène. Toutefois, d'autres huiles peuvent être utilisées dans la mesure où elles s'avèrent compatibles avec les applications visées et le complexe selon l'invention.

Un gel formé à partir du squalène et d'un complexe ou conjugué selon l'invention permet notamment d'obtenir des formulations homogènes pouvant comprendre jusqu'à au moins 3% en poids, voire au moins 10% en poids, exprimé en poids d'actif vitamine C par rapport au poids total de la composition.

Un tel gel est plus particulièrement avantageux pour administration par voie topique.

Il peut aussi se présenter sous la forme de crème, d'onguent, de pommade ou de tout autre type de formulation connue de l'homme du métier et appropriée pour les applications visées.

### Nanoparticules selon l'invention

Contre toute attente, les inventeurs ont constaté que le conjugué formé par couplage covalent d'au moins une molécule de vitamine C considérée selon l'invention avec au moins un composé hydrocarboné au sens de l'invention manifeste une aptitude à s'organiser sous une forme compactée dans un milieu solvant polaire, et conduit ainsi à la formation de nanoparticules.

Ainsi, selon un autre de ses aspects, la présente invention vise des nanoparticules du complexe conforme à l'invention.

D'une manière générale, les nanoparticules ainsi obtenues possèdent une taille moyenne variant de 30 nm à 500 nm, en particulier de 40 nm à 250 nm, voire de 45 nm à 95 nm mesurée par diffusion de la lumière à l'aide du nanosizer Coulter® N4MD, Coulter Electronics, Hialeah, USA.

Ainsi, ces particules possèdent une taille qui s'avère compatible avec tout mode d'administration, en particulier topique, injectable et oral.

Comme précisé précédemment, la formulation nanoparticulaire est avantageuse dans la mesure où elle permet notamment de formuler de manière stabilisée le complexe selon l'invention en milieu aqueux et d'amplifier la pénétration de la vitamine C jusqu'aux couches profondes de la peau.

Les nanoparticules selon l'invention sont, bien entendu, susceptibles de porter en surface une multitude de fonctions réactives, à l'image des fonctions hydroxyle ou amine par exemple. Il est donc envisageable de fixer à ces fonctions toutes sortes de molécules, notamment par des liaisons covalentes.

A titre illustratif et non limitatif de ce type de molécules susceptibles d'être associées aux nanoparticules, on peut notamment citer les molécules de type marqueur, les composés susceptibles d'assurer une fonction de ciblage, ainsi que tout composé apte à leur conférer des caractéristiques pharmacocinétiques particulières. En ce qui concerne ce dernier aspect, on peut ainsi envisager de fixer en surface de ces nanoparticules des dérivés lipophiles du polyéthylène glycol, comme par exemple le conjugué polyéthylène glycol/cholestérol, le polyéthylène glycol-phosphatidyléthano lamine ou mieux encore le polyéthylène glycol/squalène. En effet, compte-tenu de l'affinité naturelle des résidus de squalène entre eux, le conjugué polyéthylène glycol/squalène s'associe, en l'espèce, avec les nanoparticules selon l'invention, et conduit ainsi à la formation de nanoparticules revêtues en surface de polyéthylène glycol. Par ailleurs, et comme mentionné précédemment, le conjugué polyéthylène glycol/squalène agit avantageusement lors du processus de formation des nanoparticules selon l'invention, comme tensioactif du fait de son comportement amphiphile et stabilise donc la suspension colloïdale, réduisant ainsi la taille des nanoparticules formées.

Selon un mode de réalisation avantageux, les nanoparticules selon l'invention sont formulées à l'état de dispersion aqueuse.

Selon un autre mode de réalisation avantageux, les nanoparticules selon l'invention sont sous forme de lyophilisat.

De préférence, il s'agit de nanoparticules de 4,8,13,17,21-pentaméthyl-docosa-4,8,12,16,20-pentaenoate de 2-(3,4-dihydroxy-5-oxo-2,5-dihydro-furan-2-yl)-2-hydroxy-éthyle (également nommé 1,1',2-trisnor-squalenate de 2-(3,4-dihydroxy-5-oxo-2,5-dihydro-furan-2-yl)-2-hydroxy-éthyle ou acide squalénoyl-ascorbique ou encore squalénoyl-vitamine C) et préférentiellement de nanoparticules d'acide 6-squalénoyl-ascorbique (également nommé 6-squalénoyl-vitamine C).

Il peut également s'agir de préférence de nanoparticules de (5*R*)-5-[(1*S*)-1,2-dihydroxyéthyl]-4-hydroxy-3-{[(4*E*,8*E*,12*E*,16*E*)-4,8,13,17,21-pentaméthyldocosa-4,8,12,16,20-pentaèn-1-yl]oxy}-2,5-dihydrofuran-2-one, également nommé nanoparticules d'acide 2-squalényl-ascorbique ou encore nanoparticules de 2-squalényl-vitamine C.

Il peut encore également s'agir de préférence de nanoparticules de 5-(1,2-dihydroxyéthyl)-3-hydroxy-4-{[(8*E*,12*E*,16*E*)-4,8,13,17,21-pentaméthyldocosa-4,8,12,16,20-pentaèn-1-yl]oxy}-2,5-dihydrofuran-2-one, également nommé nanoparticules d'acide 3-squalényl-ascorbique ou encore nanoparticules de 3-squalényl-vitamine C.

### Procédé de préparation des nanoparticules

Plus précisément, les nanoparticules sont formées par mise en présence du conjugué avec un milieu aqueux dans des conditions propices à son agglomération à l'état de nanoparticules. Il peut notamment s'agir de méthodes dites de nanoprécipitation ou d'émulsion/évaporation de solvant.

Les nanoparticules selon la présente invention peuvent avantageusement être obtenues de la façon suivante.

Un complexe ou conjugué selon l'invention est dispersé dans au moins un solvant organique (par exemple un alcool comme l'éthanol, ou l'acétone) à une concentration suffisante pour obtenir, lors de l'ajout du mélange résultant, sous agitation, et généralement au goutte-à-goutte, à une phase aqueuse, la formation instantanée de nanoparticules selon l'invention en suspension dans ladite phase aqueuse. Le cas échéant, on procède à l'isolement desdites nanoparticules selon les techniques bien connues de l'homme du métier.

La nanoprécipitation peut généralement être réalisée à température ambiante. Quelle qu'elle soit, la température de mise en oeuvre ne doit pas affecter l'activité de la molécule de vitamine C considérée. Le procédé de préparation des nanoparticules selon l'invention est particulièrement avantageux dans la mesure où il ne requiert pas obligatoirement la présence de tensioactifs.

Cette propriété est particulièrement appréciable dans la mesure où un grand nombre de tensioactifs ne s'avèrent pas compatibles avec une application in vivo.

Ainsi, un autre avantage de la présente invention est qu'aucun solvant organique ni aucun tensioactif potentiellement toxiques ne sont nécessaires à l'élaboration desdites compositions conformes à l'invention.

Toutefois, il est entendu que l'usage de tensioactifs, généralement avantageusement dénués de toute toxicité, est envisageable dans le cadre de l'invention. Ce type de tensioactifs peut par ailleurs permettre d'accéder à des tailles encore plus réduites lors de la formation de nanoparticules. A titre illustratif et non limitatif de ce type de tensioactifs susceptibles d'être utilisés dans la présente invention, on peut notamment citer des copolymères de polyoxyéthylène-polyoxypropylène, des dérivés phospholipidiques et des dérivés lipophiles du polyéthylène glycol.

Comme dérivé lipophile du polyéthylène glycol, on peut mentionner par exemple le polyéthylène glycol cholestérol. Comme exemple des copolymères bloc polyoxyéthylène-polyoxypropylène, on peut particulièrement citer les copolymères triblocs polyoxyéthylène- polyoxypropylène- polyoxyéthylène, encore appelés poloxamères®, pluronics® ou synperonics et qui sont commercialisés, notamment, par la société BASF.

Apparentés à ces familles de copolymères, les poloxamines, qui sont constitués de segments hydrophobes (à base de polyoxypropylène), de segments hydrophiles (à base de polyoxyéthylène) et d'une partie centrale dérivant du motif éthylène diamine peuvent également être employés.

### Composition selon l'invention

Un conjugué et/ou des nanoparticules convenant à l'invention, peuvent être avantageusement formulés dans une composition pouvant se présenter sous toutes formes galéniques normalement disponibles pour le mode d'administration retenu.

Comme précisé ci-dessous, le choix de la forme considérée pour le conjugué à savoir particulaire ou non particulaire, peut être effectué en tenant compte de la nature du milieu de formulation galénique particulièrement considéré.

Ainsi, dans le cas d'une formule monophasique ou multiphasique telle une émulsion, le complexe pourra avantageusement être formulé à l'état de nanoparticules dans la phase aqueuse.

En revanche, pour une forme galénique de type crème monophasique et dédiée à être administrée par voie topique, il est avantageux de privilégier la mise en oeuvre d'une formulation du complexe selon l'invention sous la forme d'un gel huileux et plus particulièrement du complexe selon l'invention en mélange avec le squalène.

On peut envisager de formuler au moins un conjugué et/ou des nanoparticules conforme(s) à la présente invention à raison de 0,1 à 10 % en poids, exprimé en poids d'actif vitamine C, voire plus, par rapport au poids total de la composition considérée.

Une composition de l'invention comprend un milieu physiologiquement acceptable.

Par « milieu physiologiquement acceptable », on entend un milieu non toxique et susceptible d'être appliqué sur la peau et d'aspect, d'odeur et de toucher agréables.

Une composition de l'invention peut être une composition cosmétique, dermatologique, ou pharmaceutique.

De manière préférée, une composition de l'invention peut être administrée par voie topique.

Selon un mode de réalisation, une composition de l'invention administrée par voie topique peut avantageusement être formulée sous toute forme galénique convenant au soin de la peau et des muqueuses et peut se présenter sous forme d'onguent, de crèmes, de laits, de pommades, de solutions, de gels, de sprays, de lotions, ou de suspensions.

Une composition de l'invention peut également se présenter sous la forme d'un système transdermique permettant une libération active ou passive du/des nanoparticules selon l'invention par transdermie, par exemple de type patch ou gel patch (hydrogel).

Ces compositions sont préparées selon les méthodes usuelles. Les agents de formulation et excipients pour composition topique sont connus dans ces domaines et ne font pas ici l'objet d'une description détaillée.

De manière avantageuse, les compositions conformes à l'invention peuvent contenir d'autres actifs dont il peut être bénéfique de tirer profit, conjointement à l'effet de la vitamine C.

Selon un autre objet, la présente invention concerne un complément alimentaire comprenant au moins un complexe selon l'invention ou des nanoparticules selon l'invention.

La formulation des compositions pour voie orale selon l'invention peut être réalisée par tout procédé usuel connu de l'homme de l'art pour produire des solutions buvables, des dragées, des gélules, des gels, des émulsions, des comprimés à avaler ou à croquer, des capsules, notamment des capsules molles ou dures, des granulés à dissoudre, des sirops, des aliments solides ou liquides et des hydrogels.

Un conjugué selon l'invention et/ou des nanoparticules selon l'invention peuvent également être incorporés en tant que tels dans toutes formes de compléments alimentaires ou d'aliments enrichis, par exemple des barres alimentaires, des boissons lactées ou non.

Les exemples présentés ci-après sont soumis à titre illustratif et non limitatif.

### Exemples.

### Exemple la : Préparation de la 6-squalénoyl-vitamine C (VitC-SQ).

### a) Synthèse de l'acide 1,1',2-tris-nor-squalénique

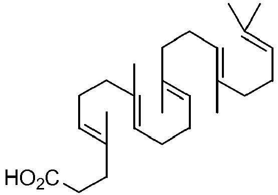

On dissout 2,77 g (7,2 mmol) d'aldéhyde 1,1',2-trisnorsqualénique (SQCHO) (Ceruti M. et al., J. Chem. Soc., Perkin Trans, 1 ; 2002, 1477-1486) dans 40 mL d'acétone. Le mélange est refroidi à 0 °C dans un bain de glace et une solution de réactif de Jones (préparée préalablement en dissolvant 26,7 g de CrO₃ dans 23 mL de H₂SO₄ concentré puis en étendant avec de l'eau jusqu'à un volume de 100 mL) est additionnée lentement jusqu'à obtention d'une coloration rouge brun persistante. Quelques gouttes d'isopropanol sont ajoutées pour décomposer l'excès de chrome (VI). Le mélange est repris dans 30 mL d'une solution aqueuse saturée en NaCl et extraite par 4x50 mL d'Et₂O. Les phases organiques sont rassemblées, lavées par 30 mL d'une solution aqueuse saturée en NaCl, séchées sur MgSO₄ puis filtrées. Les solvants sont distillés sous pression réduite pour donner une huile jaune. Le produit brut est purifié par chromatographie sur silice (Ether de pétrole/Ether diéthylique 80/20) pour donner 1,34 g d'acide trisnorsqualénique.
RMN ¹H (CDCl₃, 300 MHz) δ: 5,19-5,07 (5H, m, CH vinyliques) ; 2,45 (2H, t, *J* = 7,3 Hz, CH₂C*H*₂COOH), 2,30 (2H, t*, J* = 7,3 Hz, C*H*₂CH₂COOH), 2,09-1,98 (16H, m, CH₂ allyliques), 1,68 (3H, s, CH₃); 1,62 (3H, s, CH₃), 1,60 (12H, s, CH₃);
RMN ¹³C (CDCl₃, 75 MHz), δ: 180,0 (CO), 135,0 (C), 134,8 (2 C), 132,8 (C), 131,1 (C), 125,3 (CH), 124,4 (2 CH), 124,2 (2 CH), 39,7 (2 CH₂), 39,5 (CH₂), 34,2 (CH₂) 33,0 (CH₂), 28,2 (2 CH₂), 26,8 (CH₂), 26,6 (2 CH₂), 25,6 (CH₃), 17,6 (CH₃), 16,0 (4 CH₃).
IR (cm⁻¹): 2966, 2916, 2857, 1709, 1441, 1383, 1299, 1212, 1155, 1103;
CIMS (isobutane) m/z 401 (100);
EIMS m/z (%) : 400 (5), 357 (3), 331 (5), 289 (3), 208 (6), 136 (3), 81 (100)

### b) Synthèse du chlorure de l'acide 1,1',2-tris-nor-squalénique (ou chlorure de 4,8,13,17,21-pentaméthyl-docosa-4,8,12,16,20-pentaénoyle')

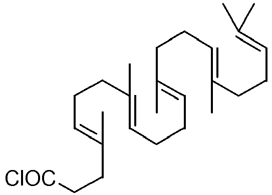

On met en solution 1,80g d'acide tris-nor-squalénique (4,5 mmol) obtenu précédemment dans 10 mL de toluène. La solution ainsi obtenue est dégazée par passage d'un courant d'azote. Puis, à cette solution est ajouté goutte à goutte à 20°C 1,2 mL de chlorure d'oxalyle (1,74 g, 13,8 mmol). Le mélange est maintenu sous agitation à température ambiante pendant 3 h.

La solution est concentrée sous pression réduite pour fournir le chlorure d'acide de l'acide 1,1',2-tris-nor-squalénique brut sous la forme d'une huile jaune.
IR (film) δ : 2920, 1799, 1443, 1382, 955, 893;
RMN ¹H (300 MHz, CDCl₃) δ (ppm): 5,23-5,10 (m, 5 H), 2,45 (t, *J* = 7,3 Hz, 2 H, CH₂C*H*₂COCl), 2,30 (t, *J* = 7,5 Hz, 2H, C*H*₂CH₂COCl), 2,15-1,95 (m, 16 H), 1,70 (s, 3H), 1,62 (s, 15H);
RMN ¹³C (75 MHz, CDCl₃), δ (ppm): 173,2 (C, COCl), 135,1 (C, *C*=CH), 134,8 (C, *C*=CH), 134,6 (C, *C*=CH), 131,4 (C, *C*=CH), 131,1 (C, *C*=CH), 126,6 (CH, C=*C*H), 124,7 (CH, C=*C*H), 124,4 (CH, C=*C*H), 124,3 (2 CH, C=*C*H), 45.8 (CH₂), 39,7 (2 CH₂), 34,4 (CH₂), 34,6 (CH₂), 28,2 (2CH₂), 26,8 (CH₂), 26,7 (CH₂), 26,5 (CH₂), 25,7 (CH₃, C=C(*C*H₃)₂), 17,6 (CH₃), 16,0 (CH₃), 15,9 (2 CH₃), 15,8 (CH₃).

### c) Synthèse de la 6-squalénoyl-vitamine C ou acide 6-O-[4,8,13,17,21-Pentaméthyl-docosa-4,8,12,16,20-pentaénoyle] ascorbique (VitC-SQ)

### c-1) Première voie (condensation avec le chlorure d'acide obtenu à l'exemple 1a)b))

Un courant d'acide chlorhydrique HCl sec est mis à barboter jusqu'à saturation dans 10 mL de N-méthylpipéridone (NMP) placés dans un flacon laveur de Durand (prise en masse).

Le solide est dissous par addition de 10 mL de NMP. 0,70 g d'acide ascorbique (4,0 mmol) sont dissous dans 10 mL de la solution obtenue précédemment et le mélange est refroidi à 0 °C. Puis, 418 mg du chlorure de l'acide préparé selon l'exemple 1a)b) (1.0 mmol) sont ajoutés et le mélange ainsi obtenu est agité à température ambiante pendant 24 h. On ajoute alors 20 mL d'eau et le mélange est extrait à l'acétate d'éthyle (4 x 20 mL). La phase organique est lavée à l'eau (2 x 5 mL), séchée sur MgSO₄ et concentrée sous pression réduite. La NMP demeurant dans le résidu est distillée sous pression réduite (0,05 mm Hg, 60 °C) pour donner un résidu épais qui est chromatographié sur gel de silice en éluant à l'acétate d'éthyle puis avec un mélange de AcOEt/MeOH, 98:2 pour fournir la 6-squalénoyl-vitamine C sous la forme d'une huile épaisse jaune pâle (190 mg, 34 %).

### c-2) Seconde voie (estérification catalysée par une lipase Novozyme 435®)

De l'acide ascorbique (352 mg, 2 mmol) est mis en suspension dans 10 mL d'alcool tert-amylique anhydre, suivis par 300 mg de Novozyme 435® (Sigma L4777), 400 mg d'acide tris-nor-squalénique préparé selon l'exemple 1a)a) (1,0 mmol) et 500 mg de tamis moléculaire 4 Å. Le mélange est placé sur un Rotavapor et est chauffé à 50 °C sous 300 mbar en agitant lentement. Après 48 h, le mélange est refroidi, filtré sur Célite et le filtrat est concentré sous pression réduite. Le résidu ainsi obtenu est chromatographié sur gel de silice en éluant à l'acétate d'éthyle puis avec un mélange AcOEt/MeOH, 98:2 pour fournir un complexe 6-squalénoyl-vitamine C sous la forme d'une huile épaisse jaune pâle (167 mg, 30 %).
[α]D = +10 (c = 0.5, EtOH) ;
IR (film) *v* : 2976, 2857, 1744, 1696, 1443, 1382,1350, 1296, 1151, 1119;
RMN 1H (400 MHz, MeOH-d4) δ (ppm): 5,20-5,00 (m, 5H), 4.68 (d, J = 1.0 Hz, 1H), 4.28-4,01 (m, 3H), 2,43 (t, J = 7,3 Hz, 2H), 2.35-2.22 (m, 2H), 2,10-1,90 (m, 16H), 1,63 (s, 3H), 1,58 (s, 3H), 1,56 (s, 12H);
RMN ¹³C (75 MHz, CDCl₃), δ (ppm): 174,6 (C, CO), 173,1(C, CO), 154,8 (C, C3), 135,9 (C, *C*=CH), 135,8 (C, *C*=CH), 135,8 (C, *C*=CH), 134,4 (C, *C*=CH), 132,0 (C, *C*=CH), 126,5 (CH, C=CH), 125,6 (CH, C=*C*H), 125,5 (CH, C=*C*H), 125,4 (2CH, C=*C*H), 120,0 (C, C2), 77.1 (CH, C4), 67,9 (CH, C5), 65,7 (CH₂, C6), 40,8 (2CH₂), 40,6 (CH₂), 35,6 (CH₂), 33,9 (CH₂), 29,2 (2CH₂), 27,8 (CH₂), 27,7 (CH₂), 27,6 (CH₂), 26,0 (CH₃, C=C(*C*H₃)₂), 17,8 (CH₃), 16,3 (3CH₃), 16,1 (CH₃);
SM (+APCI) m/z (%): 559,6 (100) [M + H].

### Exemple 1b : Préparation de l'acide 2-squalényl ascorbique (2-SO Vit C) ou (5R)-5-[(1S)-1,2-dihydroxyéthyl]-4-hydroxy-3-{[(4E,8E,12E,16E)-4,8,13,17,21-pentaméthyldocosa-4,8,12,16,20-pentaèn-1-yl]oxy}-2.5-dihydrofuran-2-one

L'acide 5,6-O-isopropylidène-L-ascorbique a été protégé sous forme d'éther de méthoxyméthyle selon la procédure de Kulkarni et Coll. (M. G. Kulkarni and S. R. Thopate, Tetrahedron, 1996, 52, 1293). Une réaction de Mitsunobu (C. Cena et al. Bioorg. Med. Chem. 2008, 16, 5199) avec le 1,1',2-trisnorsqualenol suivie d'une hydrolyse fournit l'acide 2-squalényl ascorbique (2-SQ Vit C) avec un rendement global de 32%.

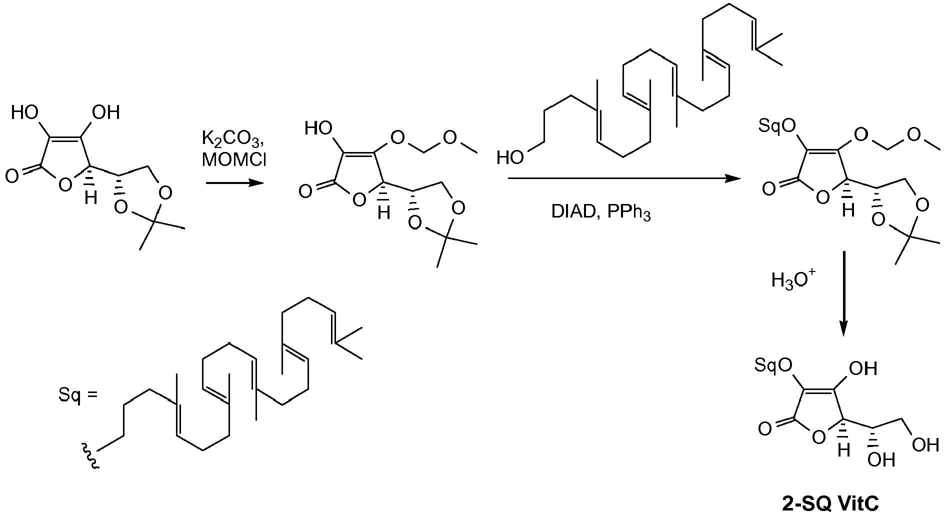

### a) (5R)-5-[(4S)-2,2-dimethyl-1,3-dioxolan-4-yl]-3-hydroxy-4-(méthoxyméthoxy)-2,5-dihydrofuran-2-one.

A une solution d'acide 5,6-isopropylidene ascorbique (6.0 g, 27.7 mmol) dans l'acétone (145 mL) sont ajoutés 3,86 g de K₂CO₃ anhydre (28 mmol) et le mélange est agité vigoureusement. 2,1 mL de chloro(méthoxy)méthane (28 mmol) dans l'acétone (10 mL) sont ajoutés goutte à goutte sur une période de deux heures. On poursuit l'agitation à reflux pendant 4 h supplémentaires. Après refroidissement, le solide est filtré et lavé avec de l'acétone. Le filtrat a été concentré sous vide et le résidu est purifié par chromatographie sur gel de silice en éluant avec un mélange cyclohexane / AcOEt / acétone 6:1:1 pour donner le composé l'éther méthoxyméthyle sous la forme d'un solide blanc (4,7 g, 65%).
¹H NMR (CDCl₃, 300 MHz) δ: 7.00-6.50 (large s, 1H, OH), 5.33 (d, *J* = 6.02 Hz, 1H, MeOC*H*₂O), 5.31 (d, *J* = 6.0 Hz, 1H, MeOC*H*₂O), 4.56 (d, *J* = 3.3 Hz, 1H, H-4), 4.26 (td, *J* = 6.7 Hz, *J* = 3.3 Hz, 1H, H-5), 4.10 (dd, *J* = 8.5, 6.8 Hz, 1H, H-6), 3.98 (dd, *J* = 8.5 Hz, *J* = 6.7 Hz, 1H, H-6), 3.54 (s, 3H, C*H*₃OCH₂), 1.30 (s, 3H, OC(C*H*₃)₂O), 1.24 (s, 3H, OC(C*H*₃)₂O).

### b) (5R)-5-[(4S)-2,2-diméthyl-1,3-dioxolan-4-yl]-4-(méthoxyméthoxy)-3-{[4E,8E,12E,16E)-4,8,13,17,21-pentaméthyldocosa-4,8,12,16,20-pentaèn-1-yl]oxyl}-2,5-dihydrofuran-2-one.

A une solution de triphénylphosphine (326 mg, 1,25 mmol) dans le THF anhydre (2 mL) refroidie à -15 °C sont ajoutés 251 mg de DIAD (1,25 mmol). Après 15 minutes, un précipité blanc de bétaïne de Mitsunobu est formé. L'agitation est poursuivie pendant 10 minutes, puis une solution de 5-(2,2-diméthyl-[1,3]dioxolan-4-yl)-3-hydroxy-4-méthoxyméthoxy-5H-furan-2-one (246 mg, 1,19 mmol) dans le THF (2 mL) est ajoutée suivie par 400 mg de 1,1',2-trisnorsqualenol (1,04 mmol) dans 2 mL de THF. Le mélange est agité à -15 ° C pendant 30 min, puis on laisse la réaction revenir à température ambiante en maintenant l'agitation pendant une période supplémentaire de 2 h. Le mélange réactionnel est alors concentré, le résidu est repris dans 20 ml d'acétate d'éthyle et la phase organique est lavée successivement par une solution saturée de bicarbonate de sodium (3 ml) puis par une solution saturée en NaCl (3 ml). Les phases organiques sont séchées sur sulfate de magnésium et concentrées sous pression réduite. Le mélange brut est purifié par flash chromatographie sur gel de silice en éluant avec un mélange cyclohexane / AcOEt (1: 1) pour donner l'acide squalényl ascorbique totalement protégé sous la forme d'une huile incolore (438 mg, 67%).
[α]_{D} +12.3 (c = 2, CHCl₃);
IR (film, cm⁻¹) 3400, 2963, 2889, 2824, 1647, 1580,1540, 1374, 1121, 1100, 1026;
¹H NMR (CDCl₃, 400 MHz) δ : 5.47 (d, *J* = 5.4 Hz, 1H, OC*H*₂OCH₃), 5.44 (d, *J* = 5.4 Hz, 1H, OC*H*₂OCH₃), 5.20-5.05 (m, 5H, =C*H*(CH₃)), 4.56 (d, *J* = 2.8 Hz, 1H, H-4), 4.32 (td, *J* = 6.7 Hz, *J* = 2.8 Hz, 1H, H-5), 4.18-4.00 (m, 4H, H-6, OC*H*₂CH₂CH₂C(CH₃)), 3.52 (s, 3H, OCH₃), 2.12-1.95 (m, 18H, =CC*H*₂C*H*₂C(CH₃)), 1.82-1.74 (m, 2H, OCH₂C*H*₂CH₂C(CH₃)), 1.68 (s, 3H, =C(C*H*₃)₂), 1.61 (s, 15H, =CH(C*H*₃)), 1.39 (s, 3H, OC(C*H*₃)₂O), 1.36 (s, 3H, OC(C*H*₃)₂O);
¹³C NMR (CDCl₃, 100 MHz) δ : 168.7 (C, C-1), 153.2 (C, C-3), 135.1 (C, =*C*(CH₃)CH₂), 135.0 (C, =*C*(CH₃)CH₂), 134.8 (C, =*C*(CH₃)CH₂), 134.0 (C, =*C*(CH₃)CH₂), 131.2 (C, =*C*(CH₃)₂), 124.8 (CH, =*C*H(CH₃), 124.4 (CH, =*C*H(CH₃), 124.3 (CH, =*C*H(CH₃), 124.2 (2CH, =*C*H(CH₃), 123.1 (C, C-2), 110.3 (C, O*C*(CH₃)₂O), 96.5 (CH₂, O*C*H₂O), 74.3 (CH, C-4), 73.8 (CH, C-5), 71.8 (CH₂, O*C*H₂CH₂CH₂C(CH₃)), 65.2 (CH₂, C-6), 57.3 (CH₃, OCH₃), 39.7 (CH₂, =C*C*H₂*C*H₂C(CH₃)), 39.6 (CH₂, =C*C*H₂*C*H₂C(CH₃)), 35.6 (CH₂, =C*C*H₂CH₂C(CH₃)), 28.3 (CH₂, =C*C*H₂*C*H₂C(CH₃)), 28.2 (CH₂, =C*C*H₂*C*H₂C(CH₃)), 26.7 (CH₂, =C*C*H₂*C*H₂C(CH₃)), 26.7 (CH₂, =C*C*H₂*C*H₂C(CH₃)), 26.6 (CH₂, =C*C*H₂*C*H₂C(CH₃)), 25.8 (CH₃, =C(*C*H₃)₂), 25.7 (CH₃, OC(C*H*₃)₂O), 25.6 (CH₃, OC(C*H*₃)₂O), 17.6 (CH₃, =CH(*C*H₃)), 16.1 (2 CH₃, =CH(*C*H₃)), 16.0 (CH₃, =CH(*C*H₃)), 15.8 (CH₃, =CH(*C*H₃));
MS (+APCI) *m*/*z* (%) : 629.7 (100) [M+H]⁺.

### c) (5R)-5-[(1S)-12-dihydroxyéthyl]-4-hydroxy-3-{[(4E,8E,12E,16E)-4,8,13,17,21-pentaméthyldocosa-4,8,12,16,20-pentaèn-1-yl]oxy}-2,5-dihydrofuran-2-one (2-SQ VitC).

A une solution du composé ci-dessus (400 mg, 0,63mmol) dans le méthanol (40 ml) on ajoute 20 mL d'une solution d'HCl 3N et le mélange résultant est agité à 50 ° C pendant 4 h. Le mélange réactionnel est alors refroidi à température ambiante et le méthanol distillé sous pression réduite. Le résidu est extrait à l'acétate d'éthyle (3 x 50 ml). Les phases organiques rassemblées sont lavées successivement par une solution saturée de bicarbonate de sodium (10 mL) puis par une solution saturée en NaCl (10 mL), séchées sur sulfate de magnésium et concentrées sous pression réduite. Le produit brut est purifié par chromatographie sur colonne de gel de silice RP-18 en éluant avec un mélange acétonitrile/eau (95/5) pour donner l'acide 2-squalényl-ascorbique (260 mg, 75 %).
[α]_{D} +13.4 (c = 2, CHCl₃);
IR (film, cm⁻¹) 3400-3100, 1700, 1650, 1588,1559, 1383, 1143, 1109, 1085 ;
¹H NMR (CD₃OD, 400 MHz) δ : 5.20-5.05 (m, 5H, =C*H*(CH₃)), 4.72 (s, 1H, H-4), 3.93 (t, *J* = 6.0 Hz, 2H, OC*H*₂CH₂CH₂C(CH₃)), 3.68 (d, *J* = 8.0 Hz, 2H, H-6), 2.12-1.94 (m, 18H, =CC*H*₂C*H*₂C(CH₃)), 1.84-1.73 (m, 2H,OCH₂C*H*₂CH₂C(CH₃)), 1.67 (s, 3H, =C(C*H*₃)₂), 1.62 (s, 3H, =CH(C*H*₃)), 1.61 (s, 6H, =CH(C*H*₃)), 1.60 (s, 6H, =CH(C*H*₃)),
¹³C NMR (CD₃OD, 100 MHz) δ : 174.0 (C, C-1), 165.1 (C, C-3), 136.2 (C, =*C*(CH₃)CH₂), 135.9 (C, =*C*(CH₃)CH₂), 135.8 (C, =*C*(CH₃)CH₂), 135.4 (C, =*C*(CH₃)CH₂), 132.0 (C, =*C*(CH₃)₂), 125.7 (CH, =*C*H(CH₃), 125.6 (CH, =*C*H(CH₃), 125.5 (CH, =*C*H(CH₃), 125.4 (2CH, =*C*H(CH₃), 121.1 (C, C-2), 77.4 (CH, C-4), 73.0 (CH₂, O*C*H₂CH₂CH₂C(CH₃)), 70.9 (CH, C-5), 63.4 (CH₂, C-6), 40.9 (2CH₂, =C*C*H₂*C*H₂C(CH₃)), 36.8 (CH₂, =C*C*H₂*C*H₂C(CH₃)), 29.2 (2CH₂, =C*C*H₂*C*H₂C(CH₃)), 27.8 (CH₂, =C*C*H₂*C*H₂C(CH₃)), 27.7 (CH₂, =C*C*H₂*C*H₂C(CH₃)), 27.6 (CH₂, =C*C*H₂*C*H₂C(CH₃)), 27.5 (CH₂, =C*C*H₂*C*H₂C(CH₃)), 25.9 (CH₃, =C(*C*H₃)₂), 17.8 (CH₃, =CH(*C*H₃)), 16.2 (3CH₃, =CH(*C*H₃)), 16.0 (CH₃, =CH(*C*H₃));
MS (-ESI) *m*/*z* (%) : 543.5 (100) [M-H]⁻.

### Exemple 1c : Préparation de l'acide 3-Squalényl-ascorbique (3-SO Vit C) ou 5-(1,2-dihydroxyéthyl)-3-hydroxy-4-{[(8E,12E,16E)-4,8,13,17,21-pentaméthyldocosa-4,8,12,16,20-pentaèn-1-yl]oxy}-2,5-dihydrofuran-2-one.

L'alkylation directe du mono anion de l'acide ascorbique par le mésylate du 1,1',2-trisnorsqualénol en utilisant le NaHCO₃ comme base dans le DMSO selon la méthode de Beifuss (U. Beifuss et al. Tetrahedron 2000, 56, 357) fournit la 3-SQ VitC avec un rendement de 37% après chromatographie sur silice RP18.

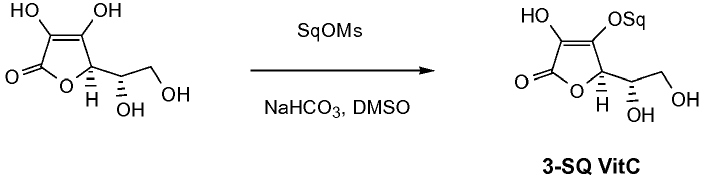

### a) Méthanesulfonate de 4,8,12,17,21,25-(4E,8E,12E,16E)-hexaméthyl-hexacosa-4,8,12,16,20,24-hexaényle.

A une solution de 1,1',2-trisnorsqualenol (475 mg, 1,23 mmol) dans le dichlorométhane (5 mL) refroidie à 0 °C, sont ajouté successivement à la seringue 205 mg de Et₃N (2,03 mol) et 177 mg de MsCl (1,54 mmol). Quelques cristaux de DMAP sont ajoutés et le mélange réactionnel est agité à 0 ° C pendant 30 min puis 1h à 20 ° C. Une solution de HCl 0,1 N (5 ml) est ajoutée et le mélange réactionnel est extrait par du CH₂Cl₂ (3 x 10 ml). Les phases organiques combinées sont lavées successivement par une solution saturée de bicarbonate de sodium (5 mL) puis par une solution saturée en NaCl (5 mL), séchées sur sulfate de magnésium et concentrées sous pression réduite fournir le mésylate de squalényle sous forme d'une huile jaune pâle (582 mg, 89%). Le mélange brut est utilisé directement dans l'étape suivante .
¹H NMR (CDCl₃, 300 MHz) δ : 5.20-5.05 (m, 5H, =C*H*(CH₃)), 4.19 (t, *J* = 7.0 Hz, 2H, C*H*₂OSO₂CH₃), 2.99 (s, 3H, SO₂C*H*₃), 2.16-1.95 (m, 18H, =CC*H*₂C*H*₂C(CH₃)), 1.88-1.75 (m, 2H, OCH₂C*H*₂CH₂C(CH₃)), 1.67 (s, 3H, =C(C*H*₃)₂), 1.60 (s, 15H, =CH(C*H*₃)).

### b) Acide 3-Squalényl-ascorbique (3-SQ VitC) ou 5-(12-dihydroxyéthyl)-3-hydroxy-4-{[(8E,12E,16E)-4,8,13,17,21-pentaméthyldocosa-4,8,12,16,20-pentaèn-1-yl]oxy}-2,5-dihydrofuran-2-one.

A une solution d'acide L-ascorbique (776 mg, 4,4 mmol) dans le DMSO anhydre (4,4 mL) on ajoute 1,29 g de NaHCO₃ (15,4 mmol) puis 600 mg de 1,1',2-trisnor-squalenylméthanesulfonate (1,26 mmol). Le mélange réactionnel est agité à 60 ° C pendant 48 h puis concentré sous pression réduite. Le résidu est traité par 2 mL de HCl 2N et extrait avec de l'acétate d'éthyle (3 X 15 ml). Les phases organiques combinées ont été séchées sur MgSO₄ et le solvant est distillé sous pression réduite. Le produit brut est purifié par chromatographie sur colonne de gel de silice RP-18 en éluant avec un mélange d'acétonitrile/H₂O (95:5) pour donner l'acide 3-squalényl-ascorbique (3-SQ Vit C) (293 mg, 37%) sous la forme d'une huile jaune pâle.
[α]_{D} +9.7 (c = 3.2, CHCl₃);
IR (film, cm⁻¹) 3400, 2950-2830, 1756, 1695, 1680, 1450, 1352, 1331, 1218, 1150, 1111;
¹H NMR (CD₃OD, 300 MHz) δ : 6.00-7.00 (large s, 1H, =C(O*H*)), 5.22-5.05 (m, 5H, =C*H*(CH₃)), 4.76 (d, *J* = 1.7 Hz, 1H, H-4), 4.55-7.40 (m, 2H, OC*H*₂CH₂CH₂C(CH₃)), 3.87 (ddd, *J* = 7,4, 5.9, 3.0 Hz, 2H, H-5), 3.72-3.85 (m, 2H, H-6), 2.15-1.95 (m, 18H, =CC*H*₂C*H*₂C(CH₃)), 1.90-1.80 (m, 2H,OCH₂C*H*₂CH₂C(CH₃)), 1.67 (s, 3H, =C(C*H*₃)₂), 1.63 (s, 3H, =CH(C*H*₃)), 1.60 (s, 12H, =CH(*C*H₃));
¹³C NMR (CDCl₃, 75 MHz) δ: 174.0 (C, C-1), 164.1 (C, C-3), 136.0 (C, =*C*(CH₃)CH₂), 135.9 (C, =*C*(CH₃)CH₂), 135.8 (C, =*C*(CH₃)CH₂), 135.6 (C, C-2), 135.4 (C, =*C*(CH₃)CH₂), 132.0 (C, =*C*(CH₃)₂), 125.8 (CH, =*C*H(CH₃), 125.6 (CH, =*C*H(CH₃), 125.5 (CH, =*C*H(CH₃), 125.4 (2CH, =*C*H(CH₃), 77.2 (CH, C-4), 73.0 (CH₂, O*C*H₂CH₂CH₂C(CH₃)), 70.8 (CH, C-5), 63.4 (CH₂, C-6), 40.9 (2CH₂, =C*C*H₂CH₂C(CH₃)), 36.9 (CH₂, =C*C*H₂CH₂C(CH₃)), 36.6 (CH₂, =C*C*H₂CH₂C(CH₃)), 29.2 (CH₂, =C*C*H₂CH₂C(CH₃)), 29.1 (CH₂, =C*C*H₂CH₂C(CH₃)), 27.8 (CH₂, =C*C*H₂CH₂C(CH₃)), 27.7 (CH₂, =C*C*H₂CH₂C(CH₃)), 27.6 (CH₂, =C*C*H₂CH₂C(CH₃)), 27.5 (CH₂, =C*C*H₂*C*H₂C(CH₃)), 25.9 (CH₃, =C(*C*H₃)₂), 17.8 (CH₃, =CH(*C*H₃)), 16.2 (3CH₃, =CH(*C*H₃)), 16.0 (CH₃, =CH(*C*H₃));
MS (+ESI) *m*/*z* (%) : 567.6 (100) [M+Na]⁺.

### Exemple 2a : Préparation des nanoparticules de 6-saualénovl-vitamine C (VitC-SQ)

Les nanoparticules sont obtenues par la méthode précipitation/évaporation de solvant, par analogie avec la méthode décrite dans Fessi et al., Int. J. Pharm., 55, 1989, R1-R4.

Une solution de 7,6 mg de vitamine C-squalénisée dans l'éthanol (0,5 mL) est ajoutée goutte à goutte à 1 mL d'une solution aqueuse (eau MilliQ®) à 3 % de Pluronic® F68 sous agitation magnétique (500 tr / min). Les particules se forment instantanément. Le flacon contenant la solution de VitC-SQ est rincé par 0,1 mL d'éthanol et la solution de rinçage est ajoutée à la suspension de nanoparticules.

Après 2 ou 3 minutes d'agitation, la suspension de nanoparticules est transférée dans un ballon de 100 mL taré et concentrée sous pression réduite au rotavapor (50-100 mbar à 20 °C pendant 10 min puis à 37 °C pendant environ 3-5 minutes) jusqu'à un poids de 0,8 - 0,9 g. La solution est alors complétée jusqu'à 1,0 g en utilisant de l'eau MilliQ®.

Dans l'eau pure, les nanoparticules de VitC-SQ sont polydispersées.

En revanche en présence de 3 % en poids de Pluronic® F68, la méthode de précipitation/évaporation de solvant fournit des nanoparticules d'une taille moyenne de 51 nm avec une faible polydispersité (0,08). La taille des nanoparticules obtenues est mesurée avec un nanosizer (Zetasizer® de la société Malvern).

### Exemple 2b : Préparation des nanoparticules de 3-squalényl-vitamine C (3-SQ VitC).

Une solution de 3-SQ Vit C (5,6 mg) dans 0,5 ml d'éthanol est ajouté goutte à goutte, sous agitation (500 t.p.m) à 1,5 mL d'eau milliQ. La précipitation des nanoparticules se produit spontanément. L'agitation est poursuivie pendant 2 ou 3 min. La suspension de nanoparticules est ensuite transférée dans un ballon à fond rond taré et l'éthanol est distillé sous pression réduite (50-100 mbar) pendant environ 10 min à température ambiante puis à 30 ° C pendant environ 3-5 min. L'évaporation est poursuivie jusqu'à un poids du contenu autour de 1,3 à 1,4 g. Le poids de la suspension est alors ajusté 1,5 g avec de l'eau milliQ ou une solution aqueuse à 5% de dextrose. Le diamètre hydrodynamique moyen des nanoparticules a été déterminé à 20 ° C par diffusion quasi-élastique de la lumière selon à un angle de 90° avec un Nanosizer de Malvern (Malvern Instruments SA, Orsay, France) dans une cuvette en quartz (longueur du trajet 10 mm). Les échantillons ont été préparés par dilution 10 fois dans l'eau de la solution mère. Le diamètre hydrodynamique moyen est donné en nombre pondéré sur toutes les populations (Z moyenne). Un diamètre moyen de 69 nm avec un indice de polydispersité de 0,22 a été observé. Les nanoparticules sont stables sur une période de trois jours.

### Exemple 3 : Préparation du gel de 6-saualénovl-vitamine C (VitC-SO) dans le squalène

Dans un pilulier de 5 mL, à 15 mg de VitC-SQ obtenu selon l'exemple la (0,027 mmol) sont ajoutés 110 mg de squalène (0,27 mmol). Le mélange est agité vigoureusement 2 min à 40 °C, puis soumis à une sonication dans une cuve à ultrason durant 1 min. Après refroidissement, on obtient un gel homogène à 3 % en poids exprimé en poids de vitamine C, soit 10 % mol en VitC-SQ.

### Exemple 4 : Evaluation de l'activité « anti-âge » du complexe de VitC-SO

Des explants de peau humaine d'un diamètre d'environ 10 nm, obtenus à partir de déchets opératoires de chirurgie esthétique et plus particulièrement issus d'une plastie abdominale de femmes âgées d'environ trente ans sont utilisés. La peau humaine est maintenue en survie dans un milieu nutritif usuel à 37°C en atmosphère humide, enrichie de 5 % de CO₂, pendant dix jours.

Les explants de peau humaine sont traités à l'aide d'un gel selon l'invention comprenant le complexe ou conjugué VitC-SQ conforme à l'invention obtenu en exemple la formulé dans du squalène comme exposé dans l'exemple 3 pour obtenir des compositions comprenant respectivement 1 % et 3 % en poids, exprimé en poids d'actif vitamine C par rapport au poids total de la composition. Pour obtenir respectivement des valeurs de 1% et 3 % exprimé en poids de vitamine C, la concentration du complexe VitC-SQ est ajustée en conséquence.

Parallèlement, trois essais témoins sont réalisés.

Le premier essai témoin est constitué d'explants de peau humaine n'ayant pas été traités (témoin N°0).

Les deux autres essais témoins mettent en oeuvre respectivement, à titre de dérivé de la vitamine C, de la vitamine C « libre » (témoin N°1) et un dérivé Vitamine C-palmitate, encore nommé ci-après VitC-palm (témoin N°2) qui peut être notamment fourni par les sociétés Sigma-Aldrich ou Alpha Aesar. Ces deux actifs sont mis en oeuvre à une concentration de 1 % et 3 % en poids, exprimé en poids d'actif vitamine C par rapport au poids total de la composition.

Un gel comprenant le dérivé VitC-Palm formulé dans du squalène est préparé selon la méthode indiquée en exemple 3 dans lequel VitC-SQ est remplacé par VitC-Palm.

Pour préparer un gel contenant de la vitamine C « libre », on introduit dans un bécher de 50 mL, 20 g d'eau distillée stérile que l'on chauffe à 50°C. Puis, 50 mg de conservateur suivis de 200 mg de vitamine C cristallisée sont ajoutés sous agitation mécanique. Par la suite, 400 mg de carboxyméthyl cellulose (notamment fourni par la société Sigma-Aldrich) sont ajoutés par petites quantités tout en évitant la formation de grumeaux. Le chauffage est stoppé et le poids initial est complété. Puis, on laisse refroidir sous agitation douce jusqu'à la formation du gel. Enfin, on transfère le gel obtenu dans un flacon hermétique.

Chacune des compositions testées est appliquée de manière topique, à raison de 2 mg par explant, sur les explants de peau humaine à l'aide d'une spatule au jour zéro (J0), puis à 1 jour (J1), à 3 jours (J3), à 6 jours (J6) et enfin à 8 jours (J8).

Au jour zéro, les explants du témoin N°0 sont prélevés et chaque explant est coupé en deux parties. Une première moitié est fixée au Bouin ordinaire et l'autre moitié est congelée à -80°C.

Au dixième jour, les explants traités avec chacune des compositions sont prélevés et sont soumis aux mêmes conditions que les explants du témoin N°0.

Après 48 heures de fixation dans le Bouin ordinaire, les prélèvements sont déshydratés et imprégnés de paraffine à l'aide d'un automate de déshydratation Leica 1020. Puis, ils sont mis en bloc selon le mode opératoire MO-H-153 à l'aide d'une station d'enrobage Leica EG 1160.

Des coupes de 5 µm sont réalisées à l'aide d'un microtome type Minot, Leica RM 2125 et sont collées sur des lames de verre histologiques silanisées superfrost.

Des observations microscopiques sont réalisées en microscopie optique, à l'aide d'un microscope Leica type Orthoplan, aux l'objectifs x 25 et x 40. Les prises de vue sont réalisées avec une caméra tri CCD Sony DXC 390P et stockées à l'aide du logiciel d'archivage de données Leica IM1000.

L'effet de chacun des actifs est caractérisé comme suit.

### a) Evaluation de l'acanthose

L'acanthose de la peau, et plus généralement l'observation de la morphologie générale est effectuée sur des coupes en paraffine après coloration au trichrome de Masson, variante de Goldner selon le mode opératoire MO-H-157.

Au temps T0, c'est-à-dire avant l'application topique des compositions, on observe que le stratum corneum est épais, très légèrement feuilleté, légèrement kératinisé en surface et à sa base.

En outre, l'épiderme présente 4 à 5 assises cellulaires avec une bonne morphologie et le relief de la jonction dermo-épidermique est net.

De plus, le derme papillaire montre des fibres de collagène épaisses formant un réseau plus ou moins dense et bien cellularisé.

Ces observations sont évidemment similaires pour les explants non traités du témoin N°0.

Au dixième jour (J10), pour les explants non traités (témoin N°0), la morphologie générale est proche de celle observée à T0.

### b) Evaluation de l'expression des GAGs.

Les GAGs neutres, réservoirs des facteurs de croissance près de la jonction dermo épidermique, et les GAGs acides, essentiellement de l'acide hyaluronique sont examinés sur coupes paraffine après coloration au bleu alcian P.A.S. (coloration de Mowry).

Avant application topique des compositions (T0) et pour ce qui est également des explants non traités (témoin N°0), on observe que les GAGs neutres sont très modérés, formant une bande rose/violacée très irrégulière et peu épaisse le long de la jonction dermo-épidermique. De plus, ils sont faibles dans le derme papillaire sous-jacent.

Par ailleurs, les GAGs acides ne sont pas modérés dans les espaces inter-cellulaires épidermiques ainsi que dans le derme papillaire.

A J10, pour les explants non traités (témoin N°0), l'expression des GAGs neutres est légèrement augmentée par rapport à celle observée à T0 et les GAGs acides sont très modérément surexprimés dans les espaces inter-cellulaires épidermiques.

Les observations, réalisées à J10 par rapport au témoin N°0, concernant les explants traités sont récapitulées dans le tableau qui suit :

| **Explants traités** | **Observations** |
|---|---|
| VitC-SQ selon l'invention à 1 % | Légère stimulation épidermique |
| | Stimulation dermique modérée |
| | Faible surexpression des GAGs neutres |
| | Aucune surexpression des GAGs acides épidermiques |
| VitC-SQ selon l'invention à 3 % | Nette stimulation épidermique |
| | Assez nette stimulation dermique |
| | Nette surexpression des GAGs neutres |
| | Aucune surexpression des GAGs acides épidermiques |
| Vitamine C libre à 1 % (Témoin N°1) | Aucune stimulation épidermique |
| | Aucune stimulation dermique |
| | Aucune surexpression des GAGs neutres |
| | Aucune surexpression des GAGs acides épidermiques |
| Vitamine C libre à 3 % (Témoin N°1) | Aucune stimulation épidermique |
| | Stimulation dermique modérée |
| | Aucune surexpression des GAGs neutres |
| | Aucune surexpression des GAGs acides épidermiques |
| VitC-palm à 1 % (Témoin N°2 ) | Aucune stimulation épidermique |
| | Aucune stimulation dermique |
| | Faible surexpression des GAGs neutres |
| | Aucune surexpression des GAGs acides épidermiques |
| VitC-palm à 3 % (Témoin N°2 ) | Aucune stimulation épidermique |
| | Stimulation dermique modérée |
| | Surexpression très modérée des GAGs neutres |
| | Aucune surexpression des GAGs acides épidermiques |

### c) Evaluation de l'expression du collagène III et du collagène I.

Pour cette appréciation, seuls certains des explants de peau humaine décrits précédemment sont utilisés. Plus particulièrement, il s'agit des explants non traités (témoin N°0) et des explants traités par VitC-SQ selon l'invention à 3 % en poids, par la Vitamine C libre à 3 % en poids (témoin N°1) et par VitC-palm à 3 % en poids (témoin N°2), exprimé en poids d'actif vitamine C par rapport au poids total de la composition.

Les observations microscopiques sont réalisées en microscopie optique, à l'aide d'un microscope Leica type DMLB, à l'objectif x 40. Les prises de vue ont été réalisées avce une caméra Olympus DP72 et à l'aide du logiciel d'acquisition et d'archivage Olympus Cel^D.

Le marquage du Collagène I est réalisé sur des coupes congelées avec un anticorps de lapin anti-collagène I humain (Monosan ref PS047, polyclonal), au 1/800^{ème} pendant 1 heure à température ambiante avec un système d'amplificateur biotine/streptavidine, révélé en fluorescence (FITC caltag SA 1001). Les noyaux ont été contre colorés à l'iodure de propidium.

Pour ce qui est du marquage du collagène III, ce dernier est marqué sur des coupes en paraffine (fixation au formol) avec un anticorps polyclonal de chèvre anti-collagène III humain (SBA ref : 1330-01), au 1/50^{ème} pendant une nuit à température ambiante à l'aide d'un système amplificateur Vectastain RTU Universal VECTOR avidine/biotine, révélé en diaminobenzidine (DAB) avec les noyaux contre colorés à l'hémalun de Masson.

Concernant le collagène I, pour les explants non traités (témoin N°0), au dixième jour, le marquage est assez net et assez dense dans tout le derme papillaire

Quant au collagène III, pour les explants témoin N°0, au dixième jour, le marquage est faible à modéré et assez régulier dans tout le derme papillaire.

Les observations, réalisées à J10 et par rapport au témoin N°0, concernant les explants traités sont récapitulées dans le tableau qui suit :

| **Explants traités** | **Observations** |
|---|---|
| VitC-SQ selon l'invention à 3 % | Légère sous expression du collagène I |
| | Légère surexpression du collagène III |
| Vitamine C libre à 3 % (Témoin N°1) | Aucune surexpression du collagène I |
| | Aucune surexpression du collagène III |
| VitC-palm à 3 % (Témoin N°2 ) | Aucune surexpression du collagène I |
| | Très légère surexpression du collagène III |

Tout d'abord, il est noté une absence d'effet de la vitamine C « libre » à une dose inférieure ou égale à 3 % en poids, exprimé en poids d'actif vitamine C par rapport au poids total de la composition.

Seul le complexe selon l'invention manifeste un effet significatif à une dose de 3 % en poids par rapport au poids total de la composition.

En conséquence, ces résultats révèlent que la présence du radical squalènoyle, amplifie contre toute attente, l'efficacité de la vitamine C jusqu'aux couches profondes de la peau puisqu'elle s'avère efficace à une concentration inférieure à la concentration usuellement requise.

Pour l'ensemble des essais réalisés à partir de 3 % en matière active, il est observé un épaississement de la peau avec une densification assez significative du collagène dans le derme papillaire. Les GAGs neutres sont également surexprimés de manière significative le long de la jonction dermo-épidermique et le collagène III est surexprimé au détriment du collagène I.

Toutefois, ces phénomènes sont significativement exacerbés avec le complexe selon l'invention.

Ainsi, il est constaté qu'à dose équivalente de vitamine C, les dérivés VitC-SQ permettent une stimulation dermique et épidermique plus importante que VitC-Palm.

En outre, il a été constaté une nette surexpression des GAGs neutres et une surexpression du collagène III au détriment du collagène I par rapport aux compositions comprenant VitC-Palm ou la vitamine C « libre ».

Par la présence d'une grande quantité de GAGs, polysaccharides ayant une forte capacité de rétention d'eau, et de collagène III dans la peau, la morphologie de celle-ci s'en trouve améliorée. La peau apparaît épaisse, de texture souple, élastique, hydratée.

Ainsi, le complexe selon l'invention présente une activité anti-âge supérieure au dérivé commercial VitC-Palm.

### Exemple 5 : Résultats sur la cytotoxicité du complexe de VitC-SO

### Test de la viabilité cellulaire (test au MTT)

10 000 cellules de fibroblastes humains CCD34SK par puits ont été ensemencées dans la plaque de 96 puits.

Les produits à tester sont ajoutés dans les puits en différentes concentrations. Après 4 h d'incubation, le milieu de culture est remplacé par nouveau milieu biologique et les cellules ont incubées pendant 48h à 37°C sous 5 % de CO₂.

Puis, le milieu de culture est éliminé par retournement et 100 µL de PBS/puits avec MTT à concentration de 0,5 mg/ml sont ajoutés. La plaque est replacée dans l'incubateur pendant 2 h à 37°C sous 5% de CO₂. Afin de dissoudre les cristaux bleu foncé de formazan formés, 100 µL d'une solution de SDS (Sodium dodécylsulfate) sont ajoutés dans chaque puits puis la plaque est ré-incubée pendant 12 h à 37°C sous 5 % de CO₂.

A l'issue de ces 12 h, les mesures spectrophotométriques sont effectuées sur un lecteur de plaques ELISA à la longueur d'onde de 570 nm. Les pourcentages de viabilité des cellules en présence des molécules à tester sont alors calculés grâce à la formule suivante : (DO moyenne des cellules traitées - DO de témoin milieu seul / DO moyenne des cellules contrôles (100 % de viabilité) - DO de témoin milieu seul) x 100 ± écart-type; ou présentés sous forme de courbes retraçant l'évolution de l'absorbance en fonction du temps.

Les CI50 ("concentration inhibitrice 50 %" soit la concentration inhibant la croissance de 50 % des cellules) correspondent alors à la concentration du produit testée engendrant une perte de 50 % dans la viabilité cellulaire (voir Tableau ci-après).

**Tableau : CI50 (µM). Activité biologique in vitro de VitC-SQ, VitC et VitC-palm sur les lignées de fibroblastes humains CCD34SK.**

| **Produit testé** | **CI50, µM** |
|---|---|
| VitC-SQ | 600 |
| VitC-palm | 250 |
| VitC | ND |

### Résultats :

A partir des résultats obtenus, la toxicité de VitC n'a pas été déterminée. Le VitC-SQ est deux fois moins toxique que le VitC-palm.

## Revendications

1. Conjugué formé d'au moins une molécule de 5-(1,2-dihydroxy-éthyl)-3,4-dihydroxy-5H-furan-2-one ou dérivé liée de manière covalente à au moins un radical hydrocarboné de formule (A) comme suit : 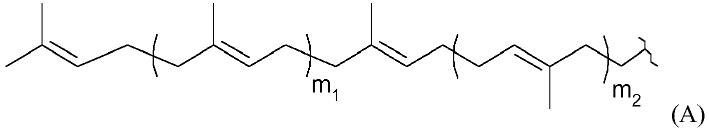 dans laquelle :
- m₁ = 0, 1, 2, 3, 4, 5 ou 6 ;
- m₂ = 0, 1,2, 3, 4, 5 ou 6 ; et
- 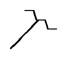 représente le site de liaison à la molécule de 5-(1,2-dihydroxy-éthyl)-3,4-dihydroxy-5H-furan-2-one ou dérivé ayant un système 3,4-dihydroxy-5H-furan-2-one tel que schématisé comme suit :

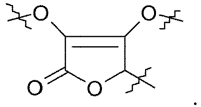

2. Conjugué selon la revendication 1, dans lequel le composé hydrocarboné comprend de 12 à 40 atomes de carbone, de préférence de 18 à 30 atomes de carbone.

3. Conjugué selon la revendication 1 ou 2, dans lequel le radical hydrocarboné est le radical de formule (A) dans lequel m₁ représente 1 et m₂ représente 2, ou m₁ représente 0 et m₂ représente 0, ou m₁ représente 1 et m₂ représente 0.

4. Conjugué selon l'une quelconque des revendications 1 à 3, de formule générale (I) 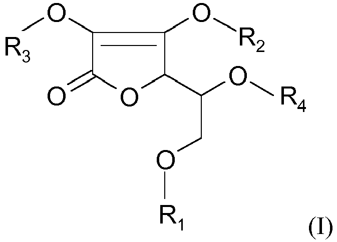 dans laquelle :
R₁, R₂, R₃ et R₄, identiques ou différents, représentent indépendamment l'un de l'autre un atome d'hydrogène ou un radical hydrocarboné de formule (A) selon l'une quelconque des revendications 1 à 3,
avec l'un au moins des groupements R₁, R₂, R₃ et R₄ étant différents de l'atome d'hydrogène.

5. Conjugué selon l'une quelconque des revendications 1 à 3, de formule générale (IA) 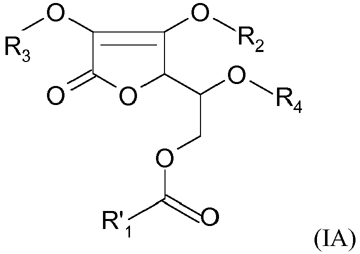 dans laquelle :
R₂, R₃ et R₄, identiques ou différents, représentent indépendamment l'un de l'autre un atome d'hydrogène ou un radical hydrocarboné de formule (A) selon l'une quelconque des revendications 1 à 3 et R'₁ représente un radical hydrocarboné de formule (A) selon l'une quelconque des revendications 1 à 3.

6. Conjugué selon la revendication 5, dans lequel R2, R3 et R4 représentent tous trois un atome d'hydrogène.

7. Procédé de préparation du conjugué selon l'une quelconque des revendications 1 à 6 comprenant :
la condensation d'au moins une molécule d'un halogénure d'acyle de formule (III) 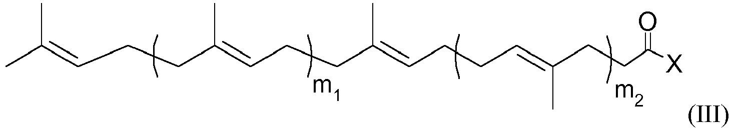 dans laquelle X est un atome d'halogène et de préférence un atome de chlore, et m₁ et m₂ sont tels que définis pour le composé de formule (A) selon l'une quelconque des revendications 1 à 3,
et d'au moins une molécule de 5-(1,2-dihydroxy-éthyl)-3,4-dihydroxy-5H-furan-2-one (IV) 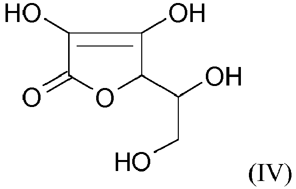 pour obtenir ledit conjugué selon l'une quelconque des revendications 1 à 6.

8. Procédé de préparation du conjugué selon l'une quelconque des revendications 1 à 6 comprenant la réaction d'estérification entre au moins une molécule de 5-(1,2-dihydroxy-éthyl)-3,4-dihydroxy-5H-furan-2-one de formule (IV) 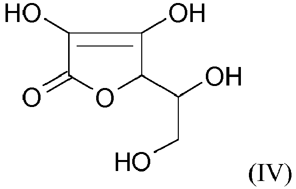 et au moins une molécule d'un acide de formule (II) 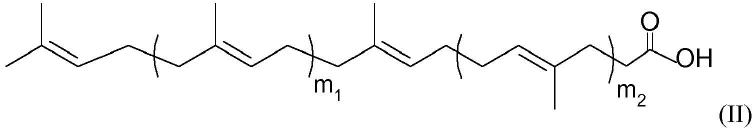 dans laquelle m₁ et m₂ sont tels que définis pour le composé de formule (A) selon l'une quelconque des revendications 1 à 3.

9. Procédé de préparation du conjugué selon l'une quelconque des revendications 1 à 6 comprenant :
la réaction entre au moins une molécule de 5-(1,2-dihydroxy-éthyl)-3,4-dihydroxy-5H-furan-2-one de formule (IV') 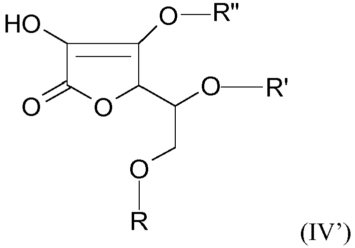 dans laquelle R, R' et R" représentent un atome d'hydrogène ou un groupement protecteur ;
et au moins une molécule d'un composé de formule (VIII) 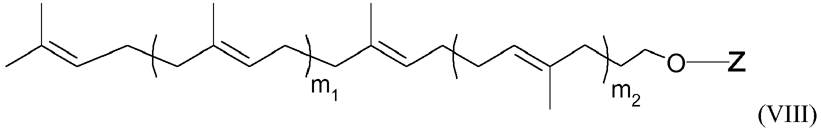 dans laquelle m₁ et m₂ sont tels que définis pour le composé de formule (A) selon l'une quelconque des revendications 1 à 3 ; et Z est un atome d'hydrogène ou un groupe -SO₂-CH₃.

10. Nanoparticules d'un conjugué tel que défini selon l'une quelconque des revendications 1 à 6.

11. Nanoparticules selon la revendication 10, **caractérisées en ce qu'**elles possèdent une taille moyenne variant de 30 nm à 500 nm, en particulier de 40 à 250 nm, voire de 45 à 95 nm.

12. Procédé de préparation de nanoparticules selon la revendication 10 ou 11, **caractérisé en ce qu'**il comprend au moins :
- la dispersion d'un conjugué selon l'une quelconque des revendications 1 à 6 dans au moins un solvant organique à une concentration suffisante pour obtenir, lors de l'ajout du mélange correspondant, sous agitation, à une phase aqueuse, la formation instantanée de nanoparticules en suspension dans ladite phase aqueuse, et, le cas échéant,
- l'isolement desdites nanoparticules.

13. Composition cosmétique, dermatologique ou alimentaire comprenant à titre de matière active au moins un conjugué selon l'une quelconque des revendications 1 à 6 et/ou des nanoparticules selon la revendication 10 ou 11 en association avec au moins un véhicule physiologiquement acceptable.

14. Utilisation cosmétique d'un conjugué selon l'une quelconque des revendications 1 à 6 et/ou de nanoparticules selon la revendication 10 ou 11 pour la prévention et/ou le traitement des signes du vieillissement de la peau du corps et/ou du visage.

15. Utilisation cosmétique d'un conjugué selon l'une quelconque des revendications 1 à 6 et/ou de nanoparticules selon la revendication 10 ou 11 pour stimuler la synthèse de collagène en particulier du collagène III et/ou stimuler la synthèse de glycosaminoglycanes.

16. Conjugué selon l'une quelconque des revendications 1 à 6 et/ou nanoparticules selon la revendication 10 ou 11 pour son utilisation en médecine.

17. Conjugué selon l'une quelconque des revendications 1 à 6 et/ou nanoparticules selon la revendication 10 ou 11 pour son utilisation dans la prévention et/ou le traitement des brûlures et/ou des plaies et/ou de toute autre pathologie liée à la cicatrisation du derme et/ou de l'épidémie.

18. Procédé de traitement cosmétique de la peau du corps et/ou du visage et/ou du cuir chevelu, dans lequel on administre une composition telle que définie dans la revendication 13.

## Patentansprüche

1. Konjugat, gebildet aus mindestens einem 5-(1,2-Dihydroxyethyl)-3,4-Dihydroxy-5H-Furan-2-on-Molekül, oder Derivat, das mit mindestens einem Kohlenwasserstoffrest der folgenden Formel (A) kovalent gebunden ist: 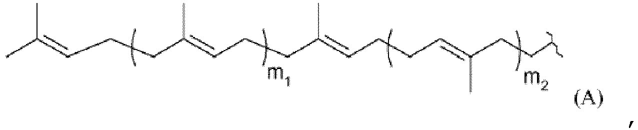 wobei:
- m₁ = 0, 1, 2, 3, 4, 5 oder 6 ;
- m₂ = 0, 1, 2, 3, 4, 5 oder 6 ; und
- 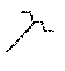 für die Bindungsstelle mit dem 5-(1,2-Dihydroxy-ethyl)-3,4-Dihydroxy-5H-Furan-2-on-Molekül steht, oder Derivat mit einer 3,4-Dihydroxy-5H-Furan-2-on-Anlage, wie folgt schematisiert:

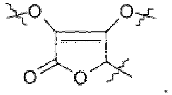

2. Konjugat nach Anspruch 1, wobei die Kohlenwasserstoffverbindung 12 bis 40 Kohlenstoffatome, vorzugsweise 18 bis 30 Kohlenstoffatome umfasst.

3. Konjugat nach Anspruch 1 oder 2, wobei der Kohlenwasserstoffrest die Gruppe der Formel (A) ist, wobei m₁ für 1 und m₂ für 2 steht oder m₁ für 0 und m₂ für 0 steht, oder m₁ für 1 und m₂ für 0 steht.

4. Konjugat nach einem der Ansprüche 1 bis 3, der allgemeinen Formel (I) 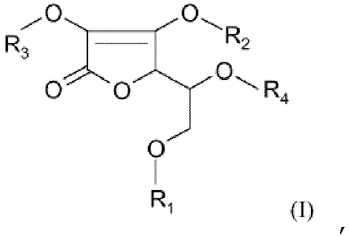 wobei:
R₁, R₂, R₃ und R₄, gleich oder verschieden, unabhängig voneinander für ein Wasserstoffatom oder einen Kohlenwasserstoffrest der Formel (A) nach einem der Ansprüche 1 bis 3 stehen,
wobei sich mindestens eine der R₁, R₂, R₃ und R₄-Gruppen vom Wasserstoffatom unterscheidet.

5. Konjugat nach einem der Ansprüche 1 bis 3, der allgemeinen Formel (IA) 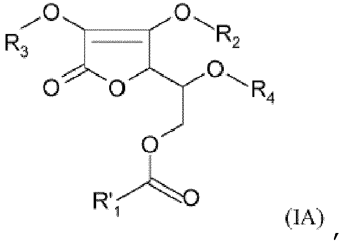 wobei:
R₂, R₃, und R₄, gleich oder verschieden, unabhängig voneinander für ein Wasserstoffatom oder einen Kohlenwasserstoffrest der Formel (A) nach einem der Ansprüche 1 bis 3 stehen und R'₁ für einen Kohlenwasserstoffrest der Formel (A) nach einem der Ansprüche 1 bis 3 steht.

6. Konjugat nach Anspruch 5, wobei R₂, R₃ und R₄ alle drei für ein Wasserstoffatom stehen.

7. Verfahren zur Herstellung eines Konjugats nach einem der Ansprüche 1 bis 6, umfassend:
die Kondensation mindestens eines Moleküls eines Acylhalogenids der Formel (III) 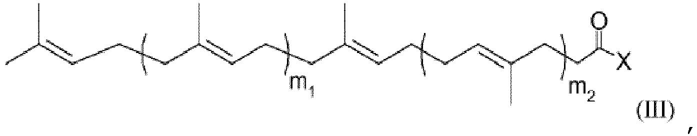 wobei X ein Halogenatom und vorzugsweise ein Chloratom ist, und m₁ und m₂ so sind wie für die Verbindung der Formel (A) nach einem der Ansprüche 1 bis 3 definiert,
und mindestens eines 5-(1,2-Dihydroxyethyl)-3,4-Dihydroxy-5H-Furan-2-on-Moleküls (IV) 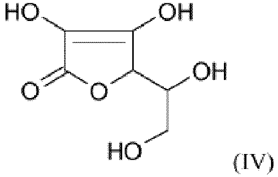 zur Gewinnung des Konjugats nach einem der Ansprüche 1 bis 6.

8. Verfahren zur Herstellung des Konjugats nach einem der Ansprüche 1 bis 6, umfassend die Veresterungsreaktion zwischen mindestens einem 5-(1,2-Dihydroxyethyl)-3,4-Dihydroxy-5H-Furan-2-on-Molekül der Formel (IV) 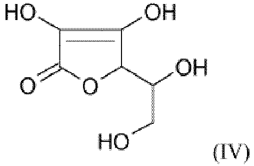 und mindestens einem Molekül einer Säure der Formel (II) 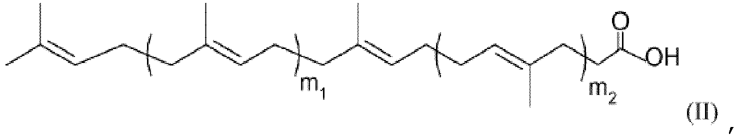 wobei m₁ und m₂ so sind wie für die Verbindung der Formel (A) nach einem der Ansprüche 1 bis 3 definiert.

9. Verfahren zur Herstellung eines Konjugats nach einem der Ansprüche 1 bis 6, umfassend:
die Reaktion zwischen mindestens einem 5-(1,2-Dihydroxyethyl)-3,4-Dihydroxy-5H-Furan-2-on-Molekül der Formel (IV') 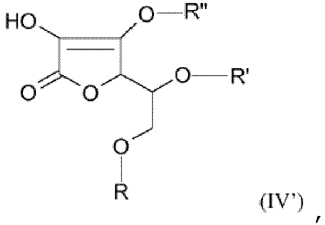 wobei R, R' und R'' für ein Wasserstoffatom oder für eine Schutzgruppe stehen, und mindestens einem Molekül einer Verbindung der Formel (VIII) 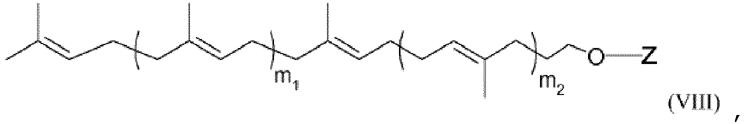 wobei m₁ und m₂ so sind wie für die Verbindung der Formel (A) nach einem der Ansprüche 1 bis 3 definiert, und Z ein Wasserstoffatom oder eine -SO₂-CH₃-Gruppe ist.

10. Nanopartikel eines Konjugats so wie nach einem der Ansprüche 1 bis 6 definiert.

11. Nanopartikel nach Anspruch 10, **dadurch gekennzeichnet, dass** sie eine durchschnittliche Größe zwischen 30 nm und 500 nm, insbesondere zwischen 40 und 250 nm beziehungsweise zwischen 45 und 95 nm haben.

12. Verfahren zur Herstellung von Nanopartikeln nach Anspruch 10 oder 11, **dadurch gekennzeichnet, dass** es mindestens umfasst:
- die Dispersion eines Konjugats nach einem der Ansprüche 1 bis 6 in mindestens einem organischen Lösungsmittel zu einer Konzentration, die ausreichend ist, um unter Rühren bei Zugabe der entsprechenden Mischung zu einer wässrigen Phase die sofortige Bildung von in der wässrigen Phase suspendierten Nanopartikeln zu erreichen, und gegebenenfalls
- die Absonderung der Nanopartikel.

13. Kosmetische, dermatologische oder Nahrungsmittelzusammensetzung mit als Wirkstoff mindestens einem Konjugat nach einem der Ansprüche 1 bis 6 und/oder Nanopartikeln nach Anspruch 10 oder 11 in Verbindung mit mindestens einem physiologisch zulässigen Vehikel.

14. Kosmetische Anwendung eines Konjugats nach einem der Ansprüche 1 bis 6 und/oder von Nanopartikeln nach Anspruch 10 oder 11 zur Vorbeugung und/oder Behandlung der Anzeichen von Körper- und/oder Gesichtshautalterung.

15. Kosmetische Anwendung eines Konjugats nach einem der Ansprüche 1 bis 6 und/oder von Nanopartikeln nach Anspruch 10 oder 11 zur Anregung der Kollagensynthese insbesondere des Kollagens III und/oder zur Anregung der Glycosaminoglycansynthese.

16. Konjugat nach einem der Ansprüche 1 bis 6 und/oder Nanopartikel nach Anspruch 10 oder 11 zu ihrer medizinischen Anwendung.

17. Konjugat nach einem der Ansprüche 1 bis 6 und/oder Nanopartikel nach Anspruch 10 oder 11 zu deren Anwendung in der Vorbeugung und/oder Behandlung von Verbrennungen und/oder Wunden und/oder von jedem anderen Krankheitsbild, das mit der Heilung der Dermis und/oder der Epidermis verbunden ist.

18. Verfahren zur kosmetischen Behandlung der Körper- und/oder Gesichts- und/oder Kopfhaut, wobei eine Zusammensetzung wie in Anspruch 13 definiert verabreicht wird.

## Claims

1. Conjugate formed of at least one molecule of 5-(1,2-dihydroxy-ethyl)-3,4-dihydroxy-5H-furan-2-one or derivative bound covalently to at least one hydrocarbon-containing radical of formula (A) as follows: 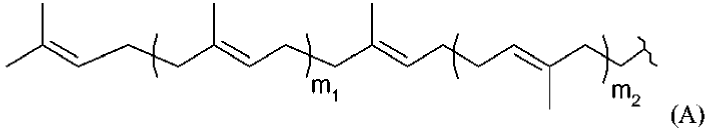 wherein:
- m₁ = 0, 1, 2, 3, 4, 5 or 6;
- m₂ = 0, 1, 2, 3, 4, 5 or 6; and
- 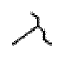 represents the binding site to the molecule of 5-(1,2-dihydroxy-ethyl)-3,4-dihydroxy-5H-furan-2-one or derivative having a 3,4-dihydroxy-5H-furan-2-one system as shown diagrammatically below:

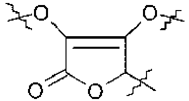

2. Conjugate according to claim 1, wherein the hydrocarbon-containing compound comprises from 12 to 40 carbon atoms, preferably from 18 to 30 carbon atoms.

3. Conjugate according to claim 1 or 2, wherein the hydrocarbon-containing radical is the radical of formula (A) in which m₁ represents 1 and m₂ represents 2, or m₁ represents 0 and m₂ represents 0, or m₁ represents 1 and m₂ represents 0.

4. Conjugate according to any one of claims 1 to 3, of general formula (I) 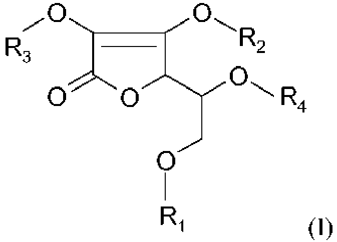 wherein:
R₁, R₂, R₃ and R₄, identical or different, represent independently of one another a hydrogen atom or a hydrocarbon-containing radical of formula (A) according to any one of claims 1 to 3,
with one at least of the R₁, R₂, R₃ and R₄ groups being different from the hydrogen atom.

5. Conjugate according to any one of claims 1 to 3, of general formula (IA) 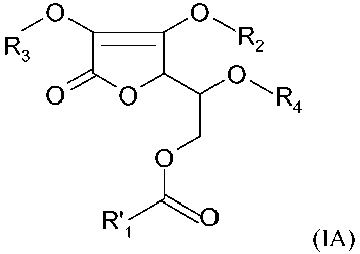 wherein:
R₂, R₃ and R₄, identical or different, represent independently of one another a hydrogen atom or a hydrocarbon-containing radical of formula (A) according to any one of claims 1 to 3 and R'₁ represents a hydrocarbon-containing radical of formula (A) according to any one of claims 1 to 3.

6. Conjugate according to claim 5, in which all three of R₂, R₃ and R₄ represent a hydrogen atom.

7. Process for the preparation of the conjugate according to any one of claims 1 to 6 comprising:
the condensation of at least one molecule of an acyl halide of formula (III) 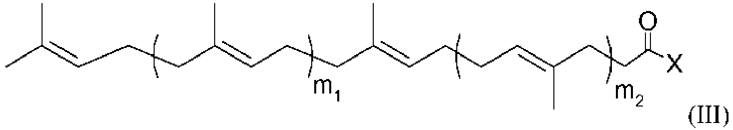 wherein X is a halogen atom and preferably a chlorine atom, and m₁ and m₂ are as defined for the compound of formula (A) according to any one of claims 1 to 3,
and at least one molecule of 5-(1,2-dihydroxy-ethyl)-3,4-dihydroxy-5H-furan-2-one (IV) 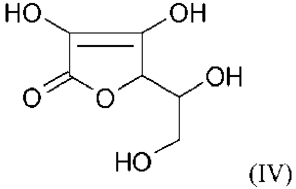 in order to obtain said conjugate according to any one of claims 1 to 6.

8. Process for the preparation of the conjugate according to any one of claims 1 to 6 comprising the esterification reaction between at least one molecule of 5-(1,2-dihydroxyethyl)-3,4-dihydroxy-5H-furan-2-one of formula (IV) 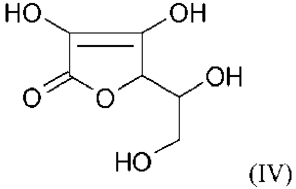 and at least one molecule of an acid of formula (II) 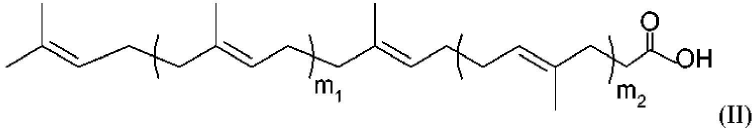 wherein m₁ and m₂ are as defined for the compound of formula (A) according to any one of claims 1 to 3.

9. Process for the preparation of the conjugate according to any one of claims 1 to 6 comprising:
the reaction between at least one molecule of 5-(1,2-dihydroxy-ethyl)-3,4-dihydroxy-5H-furan-2-one of formula (IV') 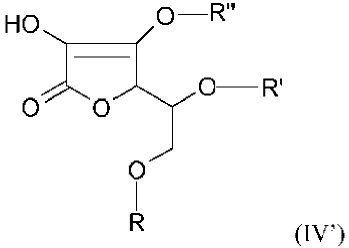 wherein R, R' and R" represent a hydrogen atom or a protecting group; and at least one molecule of a compound of formula (VIII) 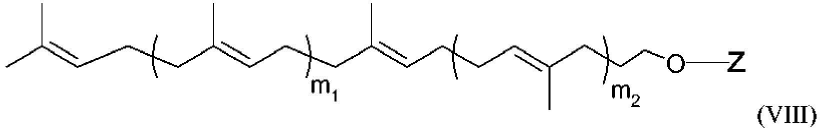 wherein m₁ and m₂ are as defined for the compound of formula (A) according to any one of claims 1 to 3; and Z is a hydrogen atom or an -SO₂-CH₃ group.

10. Nanoparticles of a conjugate as defined according to any one of claims 1 to 6.

11. Nanoparticles according to claim 10, **characterized in that** they have an average size varying from 30 nm to 500 nm, in particular from 40 to 250 nm or even from 45 to 95 nm.

12. Process for the preparation of nanoparticles according to claim 10 or 11, **characterized in that** it comprises at least:
- dispersing a conjugate according to any one of claims 1 to 6 in at least one organic solvent to a sufficient concentration to obtain, during the addition of the corresponding mixture, under stirring, to an aqueous phase, the instantaneous formation of nanoparticles in suspension in said aqueous phase, and, if appropriate,
- isolating said nanoparticles.

13. Cosmetic, dermatological or food composition comprising as active ingredient at least one conjugate according to any one of claims 1 to 6 and/or nanoparticles according to claim 10 or 11 in combination with at least one physiologically acceptable vehicle.

14. Cosmetic use of a conjugate according to any one of claims 1 to 6 and/or of nanoparticles according to claim 10 or 11 for the prevention and/or the treatment of the signs of ageing of the skin of the body and/or of the face.

15. Cosmetic use of a conjugate according to any one of claims 1 to 6 and/or of nanoparticles according to claim 10 or 11 for stimulating the synthesis of collagen in particular of collagen III and/or stimulating the synthesis of glycosaminoglycans.

16. Conjugate according to any one of claims 1 to 6 and/or nanoparticles according to claim 10 or 11 for the use thereof in medicine.

17. Conjugate according to any one of claims 1 to 6 and/or nanoparticles according to claim 10 or 11 for the use thereof in the prevention and/or the treatment of burns and/or of wounds and/or of any other pathology linked to the cicatrization of the dermis and/or the epidermis.

18. Process for the cosmetic treatment of the skin of the body and/or of the face and/or of the scalp, in which a composition as defined in claim 13 is administered.
